(19) Europäisches Patentamt
European Patent Office
Office européen des brevets

(11) EP 1 584 286 B1

(12) EUROPEAN PATENT SPECIFICATION

(45) Date of publication and mention of the grant of the patent:
**07.05.2008 Bulletin 2008/19**

(51) Int Cl.:
***A61B 5/00*** *(2006.01)* ***G06F 17/00*** *(2006.01)*

(21) Application number: **05007335.2**

(22) Date of filing: **05.04.2005**

# (54) Health management system

Gesundheitsüberwachungssystem

Système de surveillance de la santé

(84) Designated Contracting States:
**DE FR GB IT**

(30) Priority: **08.04.2004 JP 2004113748**

(43) Date of publication of application:
**12.10.2005 Bulletin 2005/41**

(73) Proprietor: **MATSUSHITA ELECTRIC INDUSTRIAL CO., LTD**
**Kadoma-shi, Osaka 571-8501 (JP)**

(72) Inventors:
- **Kawamura, Tatsurou**
  **Kyotanabe-shi**
  **Kyoto, 610-0351 (JP)**
- **Shigetoh, Nobuyuki**
  **Kyotanabe-shi**
  **Kyoto, 610-0354 (JP)**
- **Kamei, Akihito**
  **Yawata-shi**
  **Kyoto, 614-8295 (JP)**
- **Kitawaki, Fumihisa**
  **Onsen-gun**
  **Ehime, 791-0204 (JP)**

(74) Representative: **Pautex Schneider, Nicole Véronique et al**
**Novagraaf International SA**
**25, Avenue du Pailly**
**1220 Les Avanchets - Geneva (CH)**

(56) References cited:
**US-A1- 2003 125 612**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 584 286 B1

## Description

## BACKGROUND OF THE INVENTION

### (1) Field of the Invention

The present invention relates to a vital data management system and a vital data management method that support health care based on measured vital data, and especially, to a vital data utilization system and a vital data utilization method that process collected subject vital data and provide value-added information such as prediction of health condition variation.

### (2) Description of the Related Art

In order to utilize vital data for individual health care, a number of health management supporting systems having the following features have been developed: (i) obtaining subject vital data at home; (ii) sending the obtained subject vital data to a medical facility or the like; (iii) having the medical facility or the like generate value-added information by processing the subject vital data or by adding comments to the vital data in order to help the subject or a contractor understand the vital data easily; and (iv) sending the resulting value-added information to the subject or the contractor. Such a system is disclosed in the document US 2003/125612

FIG. 1 is a diagram showing the information flow of a conventional health care supporting system. In this kind of conventional health care supporting system, as shown in FIG. 1, (i) value-added information is generated by processing the vital data or by adding comments to the vital data, based on only vital data to be sent by a subject, in order to make it more understandable, and (ii) the value-added information is provided to the subject or a contractor such as his or her guardian or an employer who is directly interested with the subject (For example, refer to Japanese Laid-Open Patent application No. 2001-137199 publish). In other words, the primal feature of such a conventional health care supporting system is to notify the subject or another contractor of abnormal vital data as soon as such abnormal subject vital data has been detected, and especially to notify the subject of the present health condition accurately.

Briefly, the primal feature of the conventional health care supporting system is to help the receiver of the information grasp the present health condition. Also, the system helps the receiver judge whether an obtained vital data is abnormal or not by referring to stored past individual vital data. Therefore, the system can notify the receiver that the health condition of the specific subject is abnormal in the case where he or she gets food-poisoning or an infection, but it cannot help the receiver judge whether the abnormal health condition relates to group food-poisoning or an epidemic infection. Also, it cannot help the receiver predict how the health condition of the specific subject progresses, in other words, how the subject recovers from the illness, because databases of the other subjects corresponding to the database of the specific subject are not stored anywhere.

## SUMMARY OF THE INVENTION

The present invention is conceived considering the above-mentioned problems. A primal object of the present invention is to provide a vital data utilization system, a vital data utilization method and a vital data utilization program that are used in predicting a subject's health condition variation based on the present individual health conditions and the past individual health conditions stored in databases, and thus the invention highly contributes to the society. Also, a second object of the invention is to provide a vital data utilization system, a vital data utilization method and a vital data utilization program that are used in predicting the health condition variation of a specific subject based on his or her present health condition.

In order to achieve the above-mentioned objects, the vital data utilization system, in the present invention, stores and utilizes measured vital data. The vital data utilization system includes: a vital data measurement unit that measures first vital data of a subject; a time measurement unit that generates information on a measurement date and time at which the first vital data was measured; a subject information storage unit that stores subject information including the first vital data and the information on the measurement date and time associated with the first vital data; a reference information storage unit that stores reference information including second vital data and the information on the measurement date and time at which the second vital data was measured; a subject variation pattern generation unit that extracts, from the subject information storage unit, the subject information corresponding to a specific period that lasts until a measurement date and time of first vital data that satisfies a first predetermined condition, and that generates a subject variation pattern based on the extracted subject information, in the case where the first vital data measured by the vital data measurement unit satisfies the first predetermined condition; a reference variation pattern generation unit that extracts, from the reference information storage unit, second vital data that satisfies the first predetermined condition, that extracts, from the reference information storage unit, the reference information corresponding to the specific period that lasts until the measurement date and time at which the second vital data was measured, and that generates a reference variation pattern based on the extracted reference information; and a prediction variation pattern generation unit that compares the reference variation pattern with the subject variation pattern, and that generates a prediction variation pattern based on the reference information obtained after the reference variation pattern was generated, in the case where the comparison result satisfies a second predetermined condition.

In this way, in the case where present vital data or the vital data variation is abnormal, it is possible to (i) search, for a vital data variation pattern indicating a similar abnormal health condition, the past subject database of the subject whose health condition is abnormal or the past subject databases of the other subjects, and (ii) generate a prediction variation pattern based on a past variation pattern indicating the variation of the similar abnormal health condition.

In a first aspect of the present invention, it is preferable (i) that the vital data utilization system further includes a subject attribute information obtainment unit that obtains subject attribute information that is information on the subject, (ii) that, in the system, the subject information storage unit further stores the subject attribute information, associating with the subject information, and (iii) that the reference information storage unit stores reference attribute information associating with the reference information, the reference attribute information being information on a subject whose second vital data has obtained, and the second vital data being included in the reference information.

Also, in a second aspect of the present invention, it is preferable that the vital data utilization system further includes an order of priority assignment unit that assigns an order of priority to each prediction variation pattern generated based on each reference variation pattern, according to the comparison result of (i) the reference attribute information associated and each reference variation patterns and (ii) the subject attribute information, in the case where there are plural reference variation patterns that satisfy the second predetermined condition.

In this way, subject's attribute information such as subject sex, age, life style, and what kind of and quantity of medicine he or she is taking is included in the corresponding reference information in association with the subject variation pattern or the reference variation pattern. This makes it easier to grasp the association and specify illness based on an abnormal vital data. Further, prediction variation patterns with respective priorities are generated, which makes it easier to predict the health condition variation appropriately or judge whether the illness is epidemic or not based on such prediction variation patterns as indicators.

In a first aspect of the present invention, it is preferable (i) that the vital data utilization system further includes: subject side communication units, each of which sends the first vital data measured by the vital data measurement unit via a communication network; and a server side communication unit that sends the prediction variation patterns via the communication network, (ii) that in the system, at least the vital data measurement unit and the time measurement unit are connected to the communication network via each subject side communication unit, and at least the reference information storage unit, the reference variation pattern generation unit and the prediction variation pattern generation unit are connected to the communication network via the server side communication unit.

In this way, a number of vital data can be intensively obtained from vital data measurement units via a communication network, which makes it possible to construct a reference information storage unit that can store a lot of information, and thus it becomes possible to generate prediction variation patterns more accurately.

In a first aspect of the present invention, it is preferable that the vital data utilization system further includes a charging unit that calculates a charge and generates charging information corresponding to information amount of the generated prediction variation patterns.

Also, the vital data utilization system may further include an incentive calculation unit that calculate, on a subject-by-subject basis, incentives that are assigned to each subject who has measured his or her vital data.

Such incentives not only motivate subjects to measure vital data, but also economically help the subjects who measures vital data. This enables obtaining a large number of vital data periodically.

Also, the server in the present invention constitutes a vital data utilization system for storing and utilizing measured vital data, including: a subject information storage unit that stores subject information including the measured first vital data and information on measurement date and time at which the first vital data was measured; a reference information storage unit that stores the reference information including second vital data and the information on the measurement date and time at which the second vital data was measured; a subject variation pattern generation unit that extracts, from the subject information storage unit, the subject information corresponding to a specific period that lasts until the measurement date and time of the first vital data that satisfies a first predetermined condition, and that generates a subject variation pattern, in the case where the measured first vital data satisfies the first predetermined condition; a reference variation pattern generation unit that extracts, from the reference information storage unit, second vital information that satisfies the first predetermined condition, that extracts, from the reference information storage unit, the reference information corresponding to the specific period that lasts until the measurement date and time at which the second vital data was measured, and that generates the reference variation pattern; and a prediction variation pattern generation unit that compares the reference variation pattern with the subject variation pattern, and that generates a prediction variation pattern based on the reference information obtained after the reference variation pattern was generated in the case where the comparison result satisfies a second predetermined condition.

Note that the present invention can be realized not only as (i) a vital data utilization system like this, but also as (ii) a vital data utilization method having steps corresponding to unique units that respective apparatuses in this vital data utilization system include and as (iii) a vital data utilization program causing a computer to execute these steps.

In addition, such a program can be distributed using a recording medium such as a CD-ROM or via a communication medium such as the Internet.

## BRIEF DESCRIPTION OF THE DRAWINGS

These and other objects, advantages and features of the invention will become apparent from the following description thereof taken in conjunction with the accompanying drawings that illustrate a specific embodiment of the invention. In the Drawings:

FIG. 1 is a conceptual diagram showing a detailed structure of a conventional health care system;
FIG. 2 is a block diagram showing a structural example of a vital data utilization system in the present invention;
FIG. 3 is a block diagram showing a detailed structure of a vital data measurement unit;
FIG. 4 is a flow chart showing operations performed until vital data and the like are stored in a reference information storage unit;
FIG. 5 is a flow chart showing the operations in the vital data utilization system after the vital data and the like are stored in the reference information storage unit;
FIG. 6 is a graph displayed on an output unit, the graph being generated by synthesizing prediction variation patterns with actual measurement data;
FIG. 7 is a block diagram showing a structural example of the vital data utilization system that communicates value-added information which is generated based on the vital data via the communication network;
FIG. 8 is a flow chart showing an operation example in the vital data measurement system;
FIG. 9 is a flow chart showing an operation example of a server;
FIG. 10 is an external diagram showing how the vital data utilization system is applied to a toilet apparatus;
FIG. 11 is a graph that visually shows the latest part of the data stored in a subject information storage unit;
FIG. 12 is a graph that visually shows past data stored in a subject information storage unit;
FIG. 13 is an external diagram showing a concrete example of how the vital data measurement system is set beside a bed; and
FIG. 14 is a graph that visually shows the latest part of the data stored in a reference information storage unit.

## DESCRIPTION OF THE PREFERRED EMBODIMENT(S)

Embodiments of the vital data utilization system will be described below with reference to figures.

(First Embodiment)

FIG. 2 is a block diagram showing an outline structure of the vital data utilization system of this embodiment.

The vital data utilization system 100 shown in the figure is a stand-alone system that predicts a health condition based on a present vital data and collected vital data. The vital data utilization system 100 includes the following: (i) a vital data measurement unit 101 that measures subject vital data; (ii) a time measurement unit 102 that detects the measurement date and time at which the vital data was measured; (iii) a subject vital data storage unit 103 in which subject information including the measured vital data and the information on the measurement date and time are stored one after another; (iv) a reference information storage unit 104 to which the contents of the subject information stored in the subject information storage unit is written as reference information, by a data additionally writing unit 114; (v) a subject variation pattern generation unit 115 that extracts subject information from the subject information storage unit 103 based on a condition and generates a subject variation pattern; (vi) a reference variation pattern generation unit 116 that extracts reference information from the reference information storage unit 104 based on a condition and generates a reference variation pattern; (vii) a prediction variation pattern generation unit 105 that generates a prediction variation pattern by comparing the subject variation pattern with the reference variation pattern; and (viii) an output unit 106 that displays the output of the generated prediction variation pattern.

FIG. 3 is a block diagram showing the vital data measurement unit 101 in more detail. This vital data measurement unit 101 includes the following: (i) a measurement unit 107 that is used for obtaining the measurement values of vital data measured using a thermometer, a urine analyzer, a blood-pressure meter and the like; (ii) a vital data identification code assigning unit 108 that identifies the type of each measurement value obtained through the measurement unit 107 and assign an identification code to each measurement value; and (iii) a subject attribute information obtainment unit 117.

The subject attribute information obtainment unit 117 further includes (i) a subject identification code inputting unit 109 that assigns, to each measurement value, the information to be inputted by the subject, in order to identify the subject who has measured the vital data; and (ii) a position information assigning unit 113 that previously stores position information (residence information) such as the address of the subject or the setting place of the vital data measurement unit and assigns, to each measurement value, this position information.

The time measurement unit 102 has a function of (i) generating information on the measurement date and time at which vital data was measured through the vital data measurement unit 101, and (ii) sending, to the subject information storage unit 103, each measurement value and the information on the measurement date and time that are associated with each other.

The subject information storage unit 103 is a non-volatile memory that obtains, through a hard disc drive or the like, and holds, on a subject identification code basis, each measurement value with an assigned type code and the measurement date and time of the measurement value that are sent from the vital data measurement unit 101.

The reference information storage unit 104 holds sets of vital data and the corresponding measurement dates and time that are stored in the subject information storage unit 103, and holds the contents of the subject information that are written, by a data additionally writing unit 114, from the subject information storage unit in order to use it as reference information. Note that, likewise the subject information storage unit 103, the reference information storage unit 104 is a non-volatile memory or the like.

The subject variation pattern generation unit 115 is a processing unit for (i) obtaining the information each time latest vital data is stored in the subject information storage unit 103, and, in the case where the obtained vital data satisfies the first predetermined condition, (ii) extracting, from the subject information storage unit 103, all the subject information stored in a specific period that lasts until the latest measurement date and time, and generating a subject variation pattern indicating the variation of the subject information.

In the case where the subject variation pattern is generated by the subject variation pattern generation unit 115, the reference variation pattern generation unit 116 is a processing unit for extracting, the vital data that satisfies the first predetermined condition from among the vital data stored in the reference information storage unit 104, extracting the reference information corresponding to the same specific period as the period during which the subject variation pattern was generated and as the period that lasts until the measurement date and time at which the vital data was measured, and for generating a reference variation pattern based on the reference information.

In the case where the comparison result of the reference variation pattern and the subject variation pattern satisfies the second predetermined condition, the prediction variation pattern generation unit 105 is a processing unit for extracting, from the reference information storage unit 104, new reference information that is obtained after the latest measurement date and time shown in the reference variation pattern and that is to be used in updating the reference variation pattern, and updating the prediction variation pattern based on the new reference information.

The output unit 106 is a printer or a monitor that can visually show the prediction variation pattern. With this output unit 106, the subject can see the prediction variation pattern printed by a printer or displayed by a monitor.

The vital data identification code assigning unit 108 is a processing unit for assigning, to the measurement value received from each measurement unit 107, an identification code for identifying the type of each measurement value, with reference to a table or the like that is previously recorded.

The subject identification code inputting unit 109 includes a man-machine interface that enables the subject to input the vital data. The subject identification code inputting unit 109 assigns a code to each measurement value in order to identify the subject to which each vital data belongs, by inputting the subject ID and the like immediately before or after measuring the vital data through this subject identification code inputting unit 109.

The position information assigning unit 113 includes a storage unit composed of a non-volatile memory for holding position information (residence information) such as the address of the subject and the setting place of the vital data measurement unit. The position information assigning unit 113 assigns, to each measurement value, this position information each time new vital data is measured.

FIG. 4 is a flow chart showing the flow of the following operations performed until vital data to which the various kinds of codes are assigned or the information on the corresponding measurement dates and time are stored in the respective storage unit 103 and storage unit 104.

First, a subject identification code is inputted through the subject identification code inputting unit 109 of the subject attribute information obtainment unit 117 (S401). Next, various kinds of vital data are measured by the measurement unit 107 equipped in the vital data measurement unit 101 (S402). The time measurement unit 102 generates information on measurement date and time in response to the measurement by the measurement unit 107 (S403). The vital data identification code assigning unit 108 assigns a vital data identification code that enables identifying the type of each measured value and the vital data corresponding to the measurement value (S404). After that, the position information assigning unit 113 of the subject attribute information obtainment unit 117 assigns the position information that has been previously stored (S405). Note that this position information may be automatically obtained by the position identification system by a satellite and assigned to the measurement value. The value-added information generated based on each measurement value and the above-mentioned various information such as the corresponding information on the measurement date and time and the like are stored in an associated manner in the subject information storage unit 103 (S406). Further, the value-added information is stored in the reference information storage unit via the data additionally writing unit 114 (S407).

FIG. 5 is a flow chart showing the processing operation of generating a prediction variation pattern based on the subject information storage unit 103 and the reference information storage unit 104.

The subject information storage unit 103 holds subject information composed of sets of each measurement value and the corresponding information on the measurement date and time. The subject variation pattern generation unit 115 extracts, from this subject information storage unit 103, the latest subject information and the immediately-before subject information (S501). Also, the subject variation pattern generation unit 115 judges whether the vital data included in the subject information satisfies the first predetermined condition (S502). In the case where it is judged that the vital data satisfies the first predetermined condition (S502:Y), it extracts, from the reference information storage unit 103, all the pieces of subject information generated based on the vital data measured in a specific period that lasts until the measurement date and time, and generate the subject variation pattern indicating this subject information variation (S503).

Note that the first predetermined condition is, for example, that a body temperature that is one of vital data is at a predetermined temperature or more, or what degree per hour the latest body temperature has increased from the immediately before body temperature.

Also, a specific period is determined by totally judging whether or not the amount of vital data is enough, the vital data is reasonable and the like, and the accuracy is the trade-off of the calculation time. Also, the specific period may be the period during which a predetermined number of measurements are performed, not only a fixed period of time, day, week or the like.

Next, the reference variation pattern generation unit 116 extracts, from the reference information storage unit 104, the vital data that satisfies the first predetermined condition, extracts, from the reference information storage unit 104, all the pieces of reference information in a specific period that is the same as the period that lasts until the vital data is measured, and the reference variation pattern is generated (S504).

Next, the prediction variation pattern generation unit 105 compares the generated subject variation pattern with this reference variation pattern, and judges whether or not the comparison result of the reference variation pattern and the subject variation pattern satisfies the second predetermined condition (S505). In the case where it is judged that the second predetermined condition is satisfied (S505: Y), it extracts, from the reference information storage unit 104, new reference information obtained after the latest measurement date and time shown in the reference variation pattern and that is to be used in updating the reference variation pattern, and updating the prediction variation pattern based on the new reference information (S506).

On the other hand, in the case where no reference variation pattern that satisfies the second predetermined condition is included (S505: N), it changes the second predetermined condition (S508), and compares the reference variation pattern with the subject variation pattern based on the changed second predetermined condition.

In this embodiment, the second predetermined condition is (i) that the difference between the time variation rate of the reference variation pattern and the time variation rate of the subject variation pattern is within a predetermined range, and (ii) that the new measurement value included in the reference variation pattern is closest to the latest measurement value included in the subject variation pattern.

Also, the time variation rate is the calculation result obtained from dividing the difference between the two measurement values included in the respective variation patterns by the corresponding difference between the two measurement dates and time.

After that, it displays the generated prediction variation pattern on the output unit 106 (S507). Here, the vital data that satisfies the first predetermined condition stored in the reference information storage unit 104 is not always one. In the case where plural pieces on vital data satisfy the first predetermined condition, all the prediction variation patterns may be generated based on these pieces of reference information, or as will be described later, orders of priority are assigned to prediction variation patterns respectively based on the second predetermined condition or the other conditions, and the prediction variation patterns to which the first to a predetermined order of the priority may be assigned are displayed.

FIG. 6 shows an output example of the prediction variation pattern. In the graph, the broken line indicates a variation pattern of the vital data that have measured up to date, and the solid line indicates the generated prediction variation pattern.

In the case where an abnormal vital data is measured and the measured vital data satisfies the first predetermined condition, the vital data utilization system enables the receiver of the information to predict the variation of the health condition based on the latest subject vital data to be measured and the past subject vital data, and generates a prediction variation pattern in order to enable the receiver to confirm the prediction variation visually. In addition, this solves the subject’s anxiety and can be used for schedule making.

Note that, FIG. 6 shows an example where there is only one prediction variation pattern, but plural prediction variation patterns may be outputted. Also, such plural prediction variation patterns may be statistically processed to be displayed as a single variation prediction pattern.

Also, the second predetermined condition may be (i) that the difference between the time variation rate of the reference variation pattern and the time variation rate of the subject variation pattern is within a predetermined range, and (ii) that the new measurement time included in the reference variation pattern is closest to the latest measurement time (in this case, the comparison is made irrespective of the corresponding measurement date). In other words, in the case where the time at which the latest vital data that satisfies the first predetermined condition is 0'oclock, a prediction variation pattern is to be generated based on the reference variation pattern on condition (i) that the difference between the time variation rate of the reference variation pattern and the time variation rate of the subject variation pattern is within a predetermined range, and (ii) that the latest measurement time in the reference variation pattern is close to 0 o'clock.

(Second Embodiment)

Here will be described an embodiment of storing vital data of subjects in a reference information storage unit 104 practically in real time via the network 119, and generating prediction variation patterns with reference to variations of health conditions of the subjects. This embodiment enables effectively predicting such variations of health conditions also in the case of an epidemic infection such as the influenza. This embodiment is especially effective in the case of the influenza because symptoms vary every year.

FIG. 7 is a block diagram showing the structure of the vital data utilization system 100 in this embodiment.

Note that the units that have the same functions as the units in the first embodiment are assigned the same names and the same reference numbers respectively, and descriptions of these units will be omitted.

This vital data utilization system 100 stores, in the server 120 placed at the service provider, time variations of plural vital data and area information as attribute information of subjects in real time based on the vital data of subjects received from the plural measurement systems 110, and distributes the information analyzed based on the stored prediction variation patterns and attribute information to the service provider including the subjects.

This vital data utilization system 100 includes (i) measurement systems 110 (1) to (n), "n" being a natural number indicating how many measurement systems are set, which are respectively set at subject houses and the like, (ii) a server 120 that is set at the service provider, (iii) a personal computer 130 (called "PC" from here) that is set at the service provider, and the like. The above-mentioned measurement systems 110, the server 120, and the PC 130 are connected to each other via the communication network 119. The vital data measured through the measurement systems 110 are received by the server 120 in one time via this communication network 119, and stored practically in real time. Therefore, it is possible to generate prediction variation patterns with high accuracy and the corresponding value-added information with high availability, and distribute them.

The measurement systems 110 are set at subject houses and the like, and used in measuring vital data. They include a vital data measurement unit 101 that is a vital data measurement apparatus, a time measurement unit 102, and an output unit 106, and connected to the network 119 via the communication unit 112 that is a communication unit of the subject side.

The communication unit 112 sends, to the server 120, vital data, measurement dates and time, vital data identification codes, subject identification codes that are subject attribute information, position information, and receives value-added information such as prediction variation patterns from the server 120.

The server 120 receives and stores the vital data and the like sent from the respective measurement systems 110 in one time, and generates value-added information based on the stored information. The server 120 is realized as a computer system, and includes a communication unit 121 that is a communication unit of the server side, a data additionally writing unit 114, a providing information generation unit 123, a charging unit 124, an incentive calculation unit 125, a subject information storage unit 103, a reference information storage unit 104 and a bus 128. Note that FIG. 7 describes only primal units.

The communication unit 121 (i) receives vital data from the respective measurement systems 110 via the communication network 119, (ii) distributes prediction variation patterns generated by the providing information generation unit 123 and the corresponding value-added information, to the measurement system 110 and to the respective PC 130 that are placed at contractors.

Into the providing information generation unit 123 the earlier-mentioned subject variation pattern generation unit 115, reference variation pattern generation unit 116 and prediction variation pattern generation unit 105 are integrated. The providing information generation unit 123 analyzes prediction variation patterns and position information as attribute information corresponding to subject variation patterns and reference variation patterns, and generates value-added information. Such value-added information is, for example, the information on how many numbers of abnormal vital data are measured in a certain area.

The charging unit 124 calculates the charges for providing value-added information and generates charging information according to each user's contract coverage stored in the reference information storage unit 104. Also, the charging unit 124 updates the charging information based on the incentive information that may be generated depending on how continuously each subject sends his or her vital data.

The incentive calculation unit 125 calculates incentives to be provided to subjects who have provided vital data periodically and continuously, and generate incentive information.

Providing incentives to subjects like this promotes the subjects to measure vital data periodically and continuously and send the measurement results of vital data. Therefore, it becomes possible to collect greater number of vital data, improve the accuracy of the vital data to be stored, and enhance the effect of value-added information generated based on the vital data.

The reference information storage unit 104 is realized by a large-capacity recording medium such as a hard disc. In the reference information storage unit 104 the data additionally writing unit 114 writes, practically in real time, the vital data being stored in the subject information storage unit 103. This reference information storage unit 104 also stores subject personal information according to each subject identification code, details of contract coverage, various pieces of information used in calculating the charges. These pieces of information are updated one after another by an input operation unit (not shown in any figures) in the server 120, a charging unit 124, an incentive calculation unit 125 and the like. Note that two storage media of the subject information storage unit 103 and the reference information storage unit 104 are shown in FIG. 7. However, in reality, two storage media are not always needed, in other words, the storage units should be stored in different storage areas respectively.

Note that the PC 130 set at the contractor includes a communication unit (not shown in any figures) for receiving, from the server 120, value-added information, charging information, a receipt and the like generated based on such information. The PC 130 is connected to a monitor, a printer and the like, the monitor being for displaying the received value-added information, receipt and the like, and the printer being for printing the received value-added information, receipt and the like.

Next, the respective processing operations performed by the measurement system 110 and the server 120 will be described below.

FIG. 8 is a flow chart of the processing operations performed in the measurement system 110.

Note that the same operation steps as the steps shown in FIG. 4 are assigned the same step numbers.

First, vital data are measured using the measurement system 110 (S401 to S405). This measurement operation is the same as the operations of step 401 to step 405 in FIG. 4, step 401 inputting a subject identification code and step 405 assigning position information.

Next, the vital data and the attribute information are sent from the communication unit 112 to the server 120 via the communication network 119 (S701). The attribute information includes: information on measurement date and time generated by the time measurement unit 102; a vital data identification code generated by the vital data identification code assigning unit 108; position information generated by the position information assigning unit 113; and the subject identification code.

FIG. 9 is a flow chart indicating the processing operation of the server 120.

The information sent from the measurement system 110 is received by the communication unit (S801). The received information is stored in the subject information storage unit 103 (S406). Also, the data additionally writing unit 114 writes the received information in the reference information storage unit 104 (S407).

The incentive calculation unit 125 generates incentive information for each subject who should be provided with an incentive based on the information stored in the subject information storage unit 103, and stores the information in the reference information storage unit 104 (S802).

Next, the providing information generation unit 123 extracts, from this subject information storage unit 103, the latest subject information and the immediately-before subject information (S501), and judges whether the vital information included in the subject information satisfies the first predetermined condition (S502).

Here, the first predetermined condition is that the latest time variation rate of subject vital data is more than a certain rate.

Next, in the case where the subject variation pattern satisfies the first predetermined condition (S502: Y), all the pieces of subject information corresponding to the specific period that lasts until the measurement date and time at which the vital data was measured are extracted from the subject information storage unit 103, and the subject variation pattern indicating the variation of this subject information is generated (S503).

Next, the providing information generation unit 123 generates reference variation patterns based on the vital information that satisfies the first predetermined condition, the vital information being selected from among the vital information stored in the reference information storage unit 104 (S504). This reference variation pattern includes not only the past vital data of the subject, but also the past vital data of the other subjects.

Next, the generated subject variation pattern is compared with the reference variation pattern, and whether or not the comparison result of the reference variation pattern and the subject variation pattern satisfies the second predetermined condition is judged (S505). In the case where it satisfies the second predetermined condition (S505: Yes), new vital data obtained after reference variation patterns are generated based on the vital data that satisfy this second predetermined condition is extracted from the reference information storage unit 104, and prediction variation patterns are generated based on the vital data (S506).

On the other hand, in the case where no reference variation pattern that satisfies the second predetermined condition is included (S505: N), it changes the second predetermined condition (S508), and compares the reference variation pattern with the subject variation pattern based on the changed second predetermined condition.

Here, the second predetermined condition is that the time variation rate of the reference variation pattern is closest to the time variation rate of the subject variation pattern. This condition is determined taking into account the individual differences between a subject and the other subject, in other words, the differences based on the attribute information of respective vital data.

Next, the providing information generation unit 123 analyzes the attribute information corresponding to the reference variation patterns based on which the prediction variation patterns are generated, identifies the area where abnormal vital data are intensively collected, and generates value-added information such as position information indicating that a subject is in or near the area or is distant from the area (S803).

Next, the charging unit 124 calculates the charges and generates charging information based on the generated prediction variation patterns (804), and stores the charging information in the reference information storage unit 104.

The generated prediction variation patterns, value-added information, and charging information are sent, via the communication unit 121, to the measurement systems 110 (n) owned by subjects and the PC 130 owned by a contractor who is in relation to these subjects (S805).

The measurement systems 110 receives the prediction variation patterns and value-added information and displays the prediction variation patterns on the output unit 106 in a way that orders of priority generated by the providing information generation unit 123 are assigned to the prediction variation patterns respectively based on the value-added information. In other words, in this embodiment, the providing information generation unit 123 concurrently serves as an order of priority assignment unit. More specifically, in the case where they are displayed on the output unit 106, the more matching or similar attribute information such as address, sex, age, history of symptoms and illness are included in the vital data based on which a prediction variation pattern is generated, the order of priority becomes higher. Prediction variation patterns whose orders of priority are first to tenth are displayed on the first display screen, and the rest of prediction variation patterns whose orders of priority are eleventh and lower are displayed by scrolling the display screen. Note that, in the case where there are plural prediction variation patterns, such plural prediction variation patterns may be displayed as they are or statistically processed to be displayed as a single variation prediction pattern.

In this way, generating prediction variation patterns based on the variation patterns of the past subject vital data which is similar to the variation pattern of the present subject vital data and predicting the variation of a subject health condition based on the prediction variation patterns makes it possible to grasp the health condition variation more accurately. Also, this prediction variation patterns can be used for schedule making. This embodiment is especially effective in the case of some infections such as measles and mumps that are rarely gotten by a same person more than twice because this embodiment enables referring to the past variation patterns of the other subjects. In addition, it is possible to collect variation patterns of vital data of plural subjects in real time, and thus it becomes possible to examine position information and variations of vital data, provides and utilizes value-added information such as an indicator of the occurrence of epidemic infection in a certain area.

(First Example)

Next, a concrete example where the vital data utilization system of the present invention is applied to a toilet apparatus will be described below.

FIG. 10 is an external view showing the toilet apparatus 200 at which a vital data utilization system is set.

This toilet apparatus 200 is composed of a measurement apparatus body unit 201 and a toilet bowl 202, and realizes a stand-alone vital data utilization system.

The toilet bowl 202 includes an electrode pad 203 with a thermometer sensor that functions as a measurement unit 107 and a urine and feces taking funnel 204. The electrode pad 203 is for measuring subject electrocardiogram and body temperature and is set on the surface of the toilet bowl so that it contacts with the subject. The urine and feces taking funnel 204 is a slidable probe for sampling subject urine and feces, and is set inside the toilet bowl 202.

The measurement apparatus body unit 201 also functions as a measurement unit 107, and includes a finger insertion entrance 205, a blood testing instrument 206, a controller 207, and a control unit that is not shown in any figures.

The finger insertion entrance 205 is a hole into which a subject can insert his or her finger, and functions as a position for placing subject's finger in the measurement apparatus that is not shown and is set inside the measurement apparatus body unit 201. This measurement apparatus serves as a blood-pressure meter, a pulsimeter, and a pulsoximeter that are used for measuring blood pressure, pulse and oxygen saturation in blood through an inserted subject finger.

The blood testing instrument 206 includes an internal lancet for taking blood, and the blood testing instrument is detachable from the measurement apparatus body unit 201. The blood testing apparatus 206 has a function of measuring the number of white blood cells, C-reactive protein and the like from a very small quantity of user blood taken by driving the lancet into his or her own skin and a function of sending the measurement results to the control unit inside the measurement apparatus body unit 201 using infrared rays or a wireless communication.

The controller 207 functions as an output unit 106 that outputs and displays the prediction variation patterns and also as a subject identification code input unit 109 that inputs a subject identification code in the case where plural subjects use the vital data utilization system, and used for obtaining subject attribute information. Also, the controller 207 includes a display unit for instructing input operations to a subject and operation buttons for receiving input operations from a subject. This operation buttons include operation buttons for sliding urine and feces taking funnel 204 to set it at an appropriate position.

This toilet apparatus 200 starts measuring vital data on detecting that a subject is sat down on the toilet bowl. For example, programming that the vital data utilizing apparatus automatically starts measurement at the time when a subject urinates or defecates every morning enables causing the vital data measurement unit 101 set at the toilet bowl 202 automatically measures subject body temperature, electrocardiogram, feces viscosity, protein concentration in urine and the like, and stores the vital data as the measurement results in the subject information storage unit 103.

Setting the vital data utilization system at the toilet apparatus 200 like this enables measuring vital data at a certain date and time or when the subject health condition is stable, and also, enables preventing the subject from failing to measure vital data. This produces a merit that vital data can be obtained periodically and continuously, and information like this is very useful as reference data. Note that the measurement of vital data in the vital data measurement unit 101 can be started at the time when a subject operates the controller 207 to perform subject authentication and input the start instruction of measurement.

Next, the processing operations of the toilet apparatus 200 that serves as the vital data utilization system will be described below.

Urine and feces of a subject who sat down on the toilet bowl of the toilet apparatus 200 are measured using a urine and feces taking funnel 204, and the subject's electrocardiogram and body temperature are measured through the electrode pad 203. To simplify a description, only an example where body temperature is measured as one of vital data will be described below.

FIG. 11 is a graph indicating the time variation of subject body temperature stored in the subject information storage unit 103. In FIG. 11, S(t) indicates body temperature measured at date and time t, $T_0$ indicates the latest measurement date and time, and $T_{-1}$ indicates the measurement date and time that is older than $T_0$. For example, in the graph of FIG. 11, $S(T_{-1})$ indicates 35.5°C, and $S(T_0)$ indicates 37.0°C.

Whether or not the latest body temperature $S(T_0)$ is 36.6°C or more (in other words, whether or not the first predetermined condition is satisfied) is judged with reference to the vital data stored in the subject information storage unit 103. In the case where the latest body temperature satisfies the first predetermined condition, the vital data that have been measured in a predetermined period ($T_{-1}$ to $T_0$) are extracted from the subject information storage unit 103, and the subject variation pattern S($T_{-1}$ to $T_0$) is generated. In the graph of FIG. 11, for example, $T_0$ indicates 0 o'clock of today (0 day), and $T_{-1}$ indicates 18 o'clock of yesterday (-1 day)

Next, vital data R(Tx) of body temperature indicating 36.6°C (the first predetermined condition) is extracted from the reference information storage unit 104, and vital data that have been measured in a predetermined period (Ty to Tx) that is approximately the same length of period as the predetermined period ($T_{-1}$ to $T_0$) is extracted so as to generate the reference variation pattern R(Ty, Tx). Here, Tx is a past measurement date and time, and Ty is a measurement date and time within a specific period starting from Tx.

FIG. 12 is a graph indicating past time variation of subject body temperature stored in the reference information storage unit 104. In FIG. 12, R(t) indicates body temperature measured at a past date and time. Also, R(t)s that satisfy the condition are $R(T_{-m})$, $R(T_{-(m-1)})$ and $R(T_{-(1-1)})$. The reference variation patterns generated based on these R(t)s by extracting the vital data corresponding to the same specific period as the period to which the subject variation pattern $S(T_{-1}, T_0)$ corresponding are $R(T_{-(m+1)}, T_{-m})$, $R(T_{-m}, T_{-(m-1)})$ and $R(T_{-1}, T_{-(1-1)})$.

Next, whether or not the comparison result of the generated subject variation pattern $S(T_{-1}, T_0)$ and the reference variation pattern $R(T_y, T_x)$ satisfies the second predetermined condition is judged. In the case where it satisfies the second predetermined condition, the vital data that have been measured in the period from $T_a$ to $T_x$ is extracted from the reference information storage unit 104, the Ta being the time point earlier than $T_x$ by a certain period, and a prediction variation pattern is generated based on the vital data.

Here, the second predetermined condition is that the difference between the time variation rates of both variation patterns is the smallest. Also, the time variation rate indicates the increase rate of body temperatures that have been measured first and last in a specific period.

Note that the following condition may be set as the second predetermined condition: the difference between the time variation rates must be within a certain range, and as a result, plural reference variation patterns may be extracted.

The time variation rate Vs of body temperatures of the subject variation pattern $S(T_{-1}, T_0)$ is represented using the following expression.

$$Vs = (S(T_0) - S(T_{-1})) / (T_0 - T_{-1})) = 1.5 / 6 = 0.25℃ / \text{Time}$$

On the other hand, the resulting reference variation patterns are $R(T_{-(m+1)}, T_{-m})$, $R(T_{-m}, T_{-(m-1)})$, and $R(T_{-1}, T_{-(1-1)})$, and time variation rates of these are 0.25°C per hour, 0.5°C per hour, and 0.22°C per hour respectively. Therefore, $R(T_{-(m-1)}, T_{-m})$ which has the smallest time variation rate is selected according to the second predetermined condition.

After that, vital data that have been measured in a period that starts from $T_{-m}$ is extracted from the reference information storage unit 104 so as to generate a prediction variation pattern. For example, the time variation R(t) from 6 o'clock of the second day to present of FIG. 12 is generated as a prediction variation pattern. This prediction variation pattern indicate that the body temperature will reach the peak 6 hours later, and keep decreasing until the body temperature falls to 35.7°C that is near normal body temperature 24 hours later.

Note that the length of the period used for generating a prediction variation pattern is not restricted. For example, the period may last as long as the vital data satisfy the first predetermined condition, and a prediction variation pattern may be generated based on the time variation of the vital data.

Next, this prediction variation pattern may be synthesized into a graph shown as FIG. 11, and outputs it on the controller 207 of the toilet apparatus 200.

Note that the second predetermined condition can be the condition that the reference variation pattern generated based on the body temperature measured at the same time as time To (the date is not considered) at which the body temperature that satisfies the first predetermined condition has been measured is determined as the reference variation pattern based on which a prediction variation pattern is to be generated. In the graph of FIG. 12, $R(T_{-1}, T_{-(1+1)})$ is true of such a reference variation pattern. In this case, the time variation from 0 o'clock of the sixth day shown in the graph of FIG. 12 is determined as a prediction variation pattern. In other words, the body temperature is at the peak at present, the body temperature will keep falling to 35.5°C that is near normal body temperature 12 hours later.

Note that, the above-mentioned embodiment 1 is an example where a thermometer and an electrocardiogram are set on a part that the skin directly touches, for example, a toilet bowl of the toilet apparatus 200, but the present invention is not limited to this. For example, setting measurement instruments for measuring blood-pressure, pulse, and oxygen saturation in blood, in addition to the above-mentioned body temperature and electrocardiogram, on the part such as a toilet bowl that the skin directly touches saves the subject from having to manually measure his or her vital data. This promotes the subject to measure vital data periodically and continuously. Also, a urine analyzer for measuring glucose concentration in urine and amino-acid concentration in urine in addition to proteins in urine may be set. Further, measuring feces viscosity is effective for monitoring an infection such as food-poisoning. Also, it is recommended that albumin, globlin, hemoglobin and myoglobin as proteins in urine be measured because they are susceptible to daily variations of body conditions and thus the resulting vital data can be highly applicable. Here, the immunonephelometry is suitable for a testing method of proteins in urine. The reason is that it becomes possible to measure a specific protein (albumin, globlin, hemoglobin or the like) or hormone uniquely, and calculate the concentration of the measured component in urine. Another reason is that the measurement apparatus for performing the immunonephelometry can be easily downsized because it becomes possible to calculate the concentration by mixing urine with the antibody solution including an antibody that uniquely combines with a specific protein or hormone, and optically measuring the turbidity of the urine. In this way, as a specific protein or hormone can be measured using a comparatively small apparatus, the immunonephelometry is especially suitable for monitoring daily health conditions at home.

Also, as vital data that are especially effective for detecting an infection, the number of white blood cells in blood and C-reactive protein (CRP) concentration are listed. Also, it is possible to know the epidemic of pollinosis by measuring the number of a specific antibody (IgE-RIST) in blood.

(Second Example)

Next, a concrete example where the vital data utilization system that transmits the vital data via a communication network will be described below.

FIG. 13 is an external view indicating the status where one of the plural measurement systems 110 that is a component in the vital data utilization system is set besides a bed.

This measurement system 110 includes a finger insertion entrance 205 that functions as a measurement unit 107, a blood testing instrument 206, a controller 207, a communication cable 208 for connecting to a communication network 119, a main power switch 302, and a control unit that is not shown in any figures.

Also, this measurement system 110 also includes a measurement unit (not shown in any figures) that enables (i) measuring subject's body temperature, blood pressure, pulse, electrocardiogram, and oxygen saturation in blood during the subject is sleeping, and (ii) periodically and continuously sending the measurement results to the measurement system 110 using infrared rays or a wireless network.

FIG. 14 is a graph indicating body temperature as vital data that are stored in the reference information storage unit 104, and the graph indicates the time variation of vital data of plural subjects that have been collected via the communication network 119. In the graph, "y" that is the subscript in $R_y(t)$ is a natural variable and indicates a specific subject. Here, for example, reference signal $R_1(t)$ indicates the time variation of the subject's past body temperatures, while reference signals $R_2(t)$, $R_3(t)$ ... $R_n(t)$ indicate the time variations of the other subjects' past body temperatures.

From among those reference signals of $R_1(t)$, $R_2(t)$, $R_3(t)$ ... $R_n(t)$ the providing information generation unit 123 extracts the reference variation pattern that satisfies the second predetermined condition when comparing with the subject variation pattern.

Here, the operations of the vital data utilization system in this embodiment will be described below.

First, a subject presses a button for subject identification that is set on the controller 207 of the measurement system 110 so as to input a subject identification code that is attribute information. Next, the subject measures various vital data using measurement units 107. These measurement units 107 are set by the subject, and some of which can automatically measure vital data periodically during the subject is sleeping and send the measurement results using wireless communication.

To simplify a description, only an example where body temperature is measured as one of vital data will be described below, some parts that have been described in the first example will not be described here.

First, the latest body temperature $S(T_0)$ and the body temperature $S(T_{-1})$ that has been measured immediately before the latest one are extracted from the subject information storage unit 103, and whether or not the pattern satisfies the first predetermined condition is judged.

In the case of this example, the first predetermined condition is that the time variation rate of the body temperature has increased by 0.25°C or more per hour. In the case of the variation pattern shown in FIG. 10, since the time variation rate shown in the period from $S(T_{-1})$ to $S(T_0)$ satisfies the condition, the subject variation pattern $S(T_{-1}, T_0)$ is generated.

Next, the vital data that satisfies the first predetermined condition, that is, the vital data where body temperature has increased by 0.25°C or more per hour between the two consecutive body temperatures regarding the measurement dates and time is extracted from the reference information storage unit 104, and the reference variation pattern $R_y(T_y, T_x)$ is generated. Here, "$_y$" that is a subscript in $R_y$ is for identifying a subject, $T_x$ is a past measurement date and time, and $T_y$ is a measurement date and time that is earlier than $T_x$.

Next, the generated subject variation pattern $S(T_{-1}, T_0)$ is compared with the reference variation pattern $R_y(T_y, T_x)$, whether or not the comparison result of the reference variation pattern and the subject variation pattern satisfies the second predetermined condition is judged. In the case where the second predetermined condition is satisfied, the corresponding subject vital data that satisfies the condition and measured in the period starting from $T_x$ is extracted from the reference information storage unit 104, and a prediction variation pattern is generated based on the vital data.

After that, this prediction variation pattern may be synthesized into a graph shown as FIG. 11, and it may be outputted on a controller 207 or the like.

Here, plural reference variation patterns that satisfy the second predetermined condition may be extracted. In this case, it is preferable that orders of priority are assigned to these reference variation patterns respectively, and prediction variation patterns with orders of priority are generated based on such reference variation patterns. This is because the subject can utilize the priority in predicting the time variation of his or her own health conditions.

For example, orders of priority may be assigned based on time or attribute information. In the case of time basis, the newer the measurement date and time of vital data based on which reference variation patterns are generated is, the higher order of priority is assigned to the reference variation pattern and the corresponding prediction variation pattern. More specifically, among reference variation patterns $R_y(T_y, T_x)$, the highest order of priority is assigned to the reference variation pattern whose $T_x$ is closest to $T_0$.

On the other hand, in the case of attribute information basis, orders of priority are determined by comparing the attribute information of the subject and the attribute information corresponding to the reference variation pattern. An example of such attribute information is geographical information such as residence position information. In this case, subject's residence position information and the respectively corresponding residence position information are extracted respectively, compared with each other, and the closer to the residence of the subject based on which reference variation patterns are generated is, the higher order of priority is assigned to the reference variation pattern and the corresponding prediction variation pattern. In other words, the highest order of priority is assigned to the reference variation pattern of another subject whose residence is closest to the residence of the subject, and the more distant from the residence of the subject becomes, the lower order of priority is assigned to such reference variation pattern.

As described up to this point, assigning orders of priority based on time or attribute information such as geographical component is especially effective in the case of variations of health conditions indicating the occurrence of an infection that is epidemic in a certain area.

Likewise, it is possible to compare each subject indicating a reference variation pattern with the subject based on other types of attribute information such as age, sex, physical predisposition, physique, and history of symptoms and illness. After that, the more similar to the attribute information of the subject the attribute information based on which a reference variation pattern is generated is, the higher order of priority is assigned to such reference variation pattern. Assigning prediction variation patterns orders of priority based on such attribute information is effective because the subject can understand the reliability when predicting his or her health condition variation.

Also, it is possible to compare each subject indicating a reference variation pattern with the subject based on other types of attribute information on the subject's life style, that is, tastes and habits relating to drinking, smoking, nourishment, sleeping, and fatigue. After that, the more similar to the tastes and habits of the subject the tastes and habits based on which a reference variation pattern is generated are, the higher order of priority is assigned to such reference variation pattern. Assigning prediction variation patterns orders of priority based on such tastes and habits as attribute information is effective because the subject can understand the reliability when predicting his or her health condition variation.

Further, it is possible to compare each subject indicating a reference variation pattern with the subject based on other types of attribute information on medicine taking information. After that, the more similar to the medicine taking information the medicine taking information based on which a reference variation pattern is generated is, the higher order of priority is assigned to such reference variation pattern. Assigning prediction variation patterns orders of priority based on such medicine taking information as attribute information is effective because the subject can understand the reliability when predicting his or her health condition variation.

Also, orders of priority may be assigned to prediction variation patterns based on at least one type of attribute information among (i) information on tastes and habits and (ii) information on medicine taking. Also, orders of priority may be assigned by quantizing each type of the information included in the attribute information, assigning a weight to each type of the attribute information according to contribution, and performing multiple analysis of the attribute information.

Also, the following is more effective: not only providing prediction variation patterns to which orders of priority are assigned like described above but also (i) analyzing the relationship between the information on tastes and habits and information on medicine taking based on the reference variation pattern that is associated with at least one type of attribute information of the information on tastes and habits and the information on medicine taking and (ii) providing the subject with a piece of advice concerning what action the subject should take from now on. For example, such advice includes the information on tastes and habits and the information on medicine taking of another subject who recovers from an illness in the shortest period and whose attribute information is similar to the attribute information of the subject. The subject can utilize such advice as a guideline on how to spend a daily life in order to recover from the illness soon.

Here is an example of a subject who is in his fifties and has diabetes. In this case, from among subjects who have reference variation patterns respectively, a subject who is in his fifties and has diabetes is extracted. Say, subject 1, 2 and 3 are extracted. Subject 1 has not taken any medicine, subject 2 has taken medicine A, and subject 3 has taken medicine B. Reference variation patterns of subject 1, 2 and 3 are analyzed and the respective periods from the occurrence to recovery are judged. In the case where subject 3 has recovered in the shortest period, it is presented to a subject that a subject who has taken medicine B has recovered from the illness in the shortest period. Likewise, based on the information on tastes and habits, what tastes and habits the subject who has recovered in the shortest period have is presented to the subject.

Next, the charging unit 124 and the incentive calculation unit 125 that are set at the server 120 will be described below in detail.

The charging unit 124 generates charging information according to the information amount to be provided, for example, the number of prediction variation patterns, and the attribute information amount to be analyzed and presented.

The incentives generated by the incentive calculation unit 125 enables, for example, receiving a discount from the charge for providing value-added information, receiving a discount from the price of a test reagent, and receiving an exchange for a test reagent. Exchanging for a test reagent used by a measurement unit based on the incentive information leads to promoting measurement of vital data, and thus it is especially effective. Here, a buffer solution or an antibody solution can be used in measurement by the immunonephelometry can be listed as a test reagent.

Also, the incentive calculation unit 125 may issue the information that enables the above-mentioned exchange when the incentive information stored for each subject satisfies a predetermined condition.

Also, it is possible to provide each subject with an incentive for promoting measuring and sending vital data periodically and continuously. For example, it is possible to calculate an incentive of each subject and generate incentive information with reference to a table that is previously stored in the reference information storage unit 104, and issue an incentive that enables an exchange according to the stored incentive information. An example of providing incentives is: 20 points to the subject who has measured vital data at an interval of within one hour for three months or more; and 50 points to the subject who has measured vital data at an interval of within one hour for six months or more.

Also, it is preferable that vital data is measured frequently because the more frequently the vital data is measured, the finer subject variation patterns and reference variation patterns can be obtained, and thus the more accurate matching judgment can be made. For example, a subject may wear a wearable thermometer that is a mobile information terminal having a communication unit, always measure his or her body temperature and send the measurement value to the server 120.

Here, it is effective that the value-added information is immediately sent to the information terminal. Also, it is practical that the information terminal sends it to the server 120 only when it has generated a subject variation pattern because this saves communication amount and electric power.

Also, the incentive calculation unit 125 may issue the incentive that enables the subject to receive a 10 percent discount from the charge for providing the value-added information or the incentive that enables the subject to receive a 10 percent discount from the price of a test reagent, according to a user's selection when 20 points or more is stored for each subject. In addition, each subject may arbitrarily select one of the types of incentives through a user input.

Also, in the case where a subject selects the incentive that enables receiving a discount from the charge, the incentive information on the discount rate issued by the incentive calculation unit 125 may be notified to the charging unit 124.

Also, in the case where a subject selects the incentive that enables receiving a discount from the price of a test reagent, for example, information for issuing such a discount coupon is generated.

In the case where a subject receives a discount from the charge for providing the value-added information or a discount from the price of a test reagent, the incentive stored for the subject is decremented by the provided discount, and the incentive information in the reference information storage unit is updated.

That the incentive calculation unit 125 calculates the incentive information of each subject based on the periodicity and continuity of the measurement of vital data produces an effect that it becomes possible to collect vital data with excellent quality effectively.

Also, an incentive may be incremented based on, for example, the vital data amount for each subject stored in the reference information storage unit 104. In the case where an incentive may be incremented based on the stored vital data amount, the incentive is incremented irrespective of whether or not the measurement dates and time of the vital data are constant. However, it can promote a subject to measure vital data continuously for a long period, and it produces an effect of reducing the calculation load of the incentive calculation unit 125.

As described up to this point, a person who does not have any medical knowledge can predict health condition variation. Therefore, this prediction variation pattern is highly available in making and arranging the schedule that depends on the health condition variation. For example, in the case of having a fever caused by a cold, it is possible to estimate how many numbers of days will be needed until the fever falls and until a normal health condition is recovered, and make and arrange the schedule with reference to the estimation.

Further, it is also possible to utilize not only self vital data but also the other subjects' vital data as past vital data. In the case where a subject gets an infection such as an influenza or food-poisoning, it is possible to generate a prediction pattern with high accuracy because the subject can predict self health condition variation with reference to the health condition variation of the other subject who has gotten the same infection and has recovered from the infection.

Also, the vital data of subjects are collected in real time, and prediction variation patterns can be generated based on the vital data variations of the whole subjects. Therefore, an individual, a medical institute, a public institute, a company and the like that are the service destination of the prediction variation patterns can appropriately grasp the variation of the symptoms of an infection (for example, an influenza or food-poisoning) caused by a microbe including a virus. In this way, an individual, a medical institute, a public institute, a company and the like can take a more timely countermeasure for health management of an individual or the whole society, and further, can utilize such vital data for generating various kinds of schedules.

## Claims

**1.** A vital data utilization system (100) that stores and utilizes measured vital data, comprising:

a vital data measurement unit (101) operable to measure first vital data of a subject;
a time measurement unit (102) operable to generate information on a measurement date and time at which the first vital data was measured;
a subject information storage unit (103) operable to store subject information including the first vital data and the information on the measurement date and time associated with the first vital data;
a reference information storage unit (104) operable to store reference information including second vital data and the information on the measurement date and time at which the second vital data was measured;
a subject variation pattern generation unit (115) operable to extract, from said subject information storage unit, the subject information corresponding to a specific period that lasts until a measurement date and time of first vital data that satisfies a first predetermined condition, and operable to generate a subject variation pattern based on the extracted subject information, in the case where the first vital data measured by said vital data measurement unit satisfies the first predetermined condition;
a reference variation pattern generation unit (116) operable to extract, from said reference information storage unit, second vital data that satisfies the first predetermined condition, operable to extract, from said reference information storage unit, the reference information corresponding to the specific period that lasts until the measurement date and time at which the second vital data was measured, and operable to generate a reference variation pattern based on the extracted reference information; and
a prediction variation pattern generation unit (105) operable to compare the reference variation pattern with the subject variation pattern, and operable to generate a prediction variation pattern based on the reference information obtained after the reference variation pattern was generated, in the case where the comparison result satisfies a second predetermined condition.

**2.** The vital data utilization system according to Claim 1, further comprising
a subject attribute information obtainment unit (117) operable to obtain subject attribute information that is information on the subject,
wherein said subject information storage unit (103) is further operable to store the subject attribute information, associating with the subject information, and
said reference information storage unit (104) is operable to store reference attribute information associating with the reference information, the reference attribute information being information on a subject whose second vital data has obtained, and the second vital data being included in the reference information.

**3.** The vital data utilization system according to Claim 2, further comprising
an order of priority assignment unit (123) operable to assign an order of priority to each prediction variation pattern generated based on each reference variation pattern, according to the comparison result of (i) the reference attribute information associated with each reference variation patterns and (ii) the subject attribute information, in the case where there are plural reference variation patterns that satisfy the second predetermined condition.

**4.** The vital data utilization system according to Claim 1, further comprising:

subject side communication units (112), each of which is operable to send the first vital data measured by said vital data measurement unit (101) via a communication network (119); and
a server side communication unit (121) operable to send the prediction variation patterns via the communication network,
wherein at least said vital data measurement unit and said time measurement unit (102) are connected to the communication network via each subject side communication unit, and
at least said reference information storage unit (104), said reference variation pattern generation unit (115) and said prediction variation pattern generation unit (105) are connected to the communication network via said server side communication unit.

**5.** The vital data utilization system according to Claim 1, further comprising
a charging unit (124) operable to calculate a charge and generate charging information corresponding to information amount of the generated prediction variation patterns.

**6.** The vital data utilization system according to Claim 1, further comprising
an incentive calculation unit (125) operable to calculate, on a subject-by-subject basis, an incentive and operable to generate incentive information indicating that each subject has measured vital data.

**7.** A server (120) that constitutes a vital data utilization system (100) for storing and utilizing measured vital data, comprising:

a subject information storage unit (103) operable to store subject information including the measured first vital data and information on measurement date and time at which the first vital data was measured;
a reference information storage unit (104) operable to store the reference information including second vital data and the information on the measurement date and time at which the second vital data was measured;
a subject variation pattern generation unit (115) operable to extract, from said subject information storage unit, the subject information corresponding to a specific period that lasts until the measurement date and time of first vital data that satisfies a first predetermined condition, and operable to generate a subject variation pattern based on the extracted subject information, in the case where the measured first vital data satisfies the first predetermined condition;
a reference variation pattern generation unit (116) operable to extract, from said reference information storage unit, second vital information that satisfies the first predetermined condition, operable to extract, from said reference information storage unit, the reference information corresponding to the specific period that lasts until the measurement date and time at which the second vital data was measured, and operable to generate the reference variation pattern based on the extracted reference information; and
a prediction variation pattern generation unit (105) operable to compare the reference variation pattern with the subject variation pattern, and operable to generate a prediction variation pattern based on the reference information obtained after the reference variation pattern was generated in the case where the comparison result satisfies a second predetermined condition.

**8.** A vital data utilization method for storing and utilizing measured vital data, comprising:

a vital data measurement step of measuring first vital data of a subject;
a time measurement step of generating information on measurement date and time at which the first vital data was measured;
a subject information storage step of storing, in a subject information storage unit, subject information including the first vital data and the information on the measurement date and time associated with the first vital data;
a reference information storage step of storing, in a reference information storage unit, the reference information including second vital data and the measurement date and time at which the second vital data was measured;
a first judgment step of judging whether or not the measured first vital data satisfies the first predetermined condition;
a subject variation pattern generation step of extracting, from the subject information storage unit, the subject information corresponding to a specific period that lasts until the measurement date and time of the first vital data that satisfies the first predetermined condition, and generating the subject variation pattern based on the extracted subject information, in the case where it is judged in said first judgment step that the first vital data satisfies the first predetermined condition;
a reference variation pattern generation step of extracting, from the reference information storage unit, second vital data that satisfies the first predetermined condition, extracting, from the reference information storage unit, the reference information corresponding to the specific period that lasts until the measurement date and time at which the second vital data was measured, and generating a reference variation pattern; and
a prediction variation pattern generation step of comparing the reference variation pattern with the subject variation pattern, and generating a prediction variation pattern based on the reference information obtained after the reference variation pattern was generated based on the extracted reference information, in the case where the comparison result satisfies a second predetermined condition.

**9.** A vital data utilization program causing a computer to execute a method for storing and utilizing measured vital data, the program comprising:

a vital data measurement step of measuring first vital data of a subject;
a time measurement step of generating information on measurement date and time at which the first vital data was measured;
a subject information storage step of storing, in a subject information storage unit, subject information including the first vital data and the information on the measurement date and time associated with the first vital data;
a reference information storage step of storing, in a reference information storage unit, the reference information including second vital data and the information on the measurement date and time at which the second vital data was measured;
a first judgment step of judging whether or not the measured first vital data satisfies the first predetermined condition;
a subject variation pattern generation step of extracting, from the subject information storage unit, the subject information corresponding to a specific period that lasts until the measurement date and time of the first vital data that satisfies the first predetermined condition, and generating the subject variation pattern based on the extracted subject information, in the case where it is judged in said first judgment step that the first vital data satisfies the first predetermined condition;
a reference variation pattern generation step of extracting, from the reference information storage unit, second vital data that satisfies the first predetermined condition, extracting, from the reference information storage unit, the reference information corresponding to the specific period that lasts until the measurement date and time at which the second vital data was measured, and generating a reference variation pattern; and
a prediction variation pattern generation step of comparing the reference variation pattern with the subject variation pattern, and generating a prediction variation pattern based on the reference information obtained after the reference variation pattern was generated based on the extracted reference information, in the case where the comparison result satisfies a second predetermined condition.

**Patentansprüche**

1. Lebenswichtige-Daten-Verwertungssystem (100), das gemessene lebenswichtige Daten speichert und verwertet, mit:

   einer Lebenswichtige-Daten-Messeinheit (101), die so betreibbar ist, dass sie erste lebenswichtige Daten einer Person misst;
   einer Zeitmesseinheit (102), die so betreibbar ist, dass sie Informationen zu einem Messdatum und einer Messzeit erzeugt, an dem / zu der die ersten lebenswichtigen Daten gemessen wurden;
   einer Personeninformationen-Speichereinheit (103), die so betreibbar ist, dass sie Personeninformationen speichert, die die ersten lebenswichtigen Daten und die mit den ersten lebenswichtigen Daten assoziierten Informationen zu dem Messdatum und der Messzeit enthalten;
   einer Referenzinformationen-Speichereinheit (104), die so betreibbar ist, dass sie Referenzinformationen speichert, die zweite lebenswichtige Daten und die Informationen zu dem Messdatum und der Messzeit enthalten, an dem / zu der die zweiten lebenswichtigen Daten gemessen wurden;
   einer Personenänderungsmuster-Erzeugungseinheit (115), die so betreibbar ist, dass sie aus der Personeninformationen-Speichereinheit die Personeninformationen extrahiert, die einem bestimmten Zeitraum entsprechen, der bis zu einem Messdatum und einer Messzeit der ersten lebenswichtigen Daten reicht, die eine erste vorgegebene Bedingung erfüllen, und so betreibbar ist, dass sie aufgrund der extrahierten Personeninformationen ein Personenänderungsmuster erzeugt, wenn die von der Lebenswichtige-Daten-Messeinheit gemessenen ersten lebenswichtigen Daten die erste vorgegebene Bedingung erfüllen;
   einer Referenzänderungsmuster-Erzeugungseinheit (116), die so betreibbar ist, dass sie aus der Referenzinformationen-Speichereinheit zweite lebenswichtige Daten extrahiert, die die erste vorgegebene Bedingung erfüllen, so betreibbar ist, dass sie aus der Referenzinformationen-Speichereinheit die Referenzinformationen extrahiert, die dem bestimmten Zeitraum entsprechen, der bis zu dem Messdatum und der Messzeit reicht, an dem / zu der die zweiten lebenswichtigen Daten gemessen wurden, und so betreibbar ist, dass sie aufgrund der extrahierten Referenzinformationen ein Referenzänderungsmuster erzeugt; und
   einer Prädiktionsänderungsmuster-Erzeugungseinheit (105), die so betreibbar ist, dass sie das Referenzänderungsmuster mit dem Personenänderungsmuster vergleicht, und so betreibbar ist, dass sie aufgrund der Referenzinformationen, die nach dem Erzeugen des Referenzänderungsmusters gewonnen worden sind, ein Prädiktionsänderungsmuster erzeugt, wenn das Vergleichsergebnis eine zweite vorgegebene Bedingung erfüllt.

2. Lebenswichtige-Daten-Verwertungssystem nach Anspruch 1, das weiterhin eine Personenattributinformationen-Gewinnungseinheit (117) aufweist, die so betreibbar ist, dass sie Personenattributinformationen, das heißt, Informationen zu der Person, gewinnt,
   **dadurch gekennzeichnet, dass**
   die Personeninformationen-Speichereinheit (103) weiterhin so betreibbar ist, dass sie die Personenattributinformationen durch Assoziieren mit den Personeninformationen speichert, und
   die Referenzinformationen-Speichereinheit (104) so betreibbar ist, dass sie Referenzattributinformationen durch Assoziieren mit den Referenzinformationen speichert, wobei die Referenzattributinformationen Informationen zu einer Person sind, deren zweite lebenswichtige Daten gewonnen worden sind, und die zweiten lebenswichtigen Daten in den Referenzinformationen enthalten sind.

3. Lebenswichtige-Daten-Verwertungssystem nach Anspruch 2, das weiterhin eine Prioritätenreihenfolge-Zuweisungseinheit (123) aufweist, die so betreibbar ist, dass sie jedem Prädiktionsänderungsmuster, das aufgrund jedes Referenzänderungsmusters erzeugt wird, entsprechend dem Vergleichsergebnis (I) der Referenzattributinformationen, die mit den einzelnen Referenzänderungsmustern assoziiert sind, und (II) der Personenattributinformationen, wenn es mehrere Referenzänderungsmuster gibt, die die zweite vorgegebene Bedingung erfüllen, eine Prioritätenreihenfolge zuweist.

4. Lebenswichtige-Daten-Verwertungssystem nach Anspruch 1, das weiterhin Folgendes aufweist:

personenseitige Kommunikationseinheiten (112), die jeweils so betreibbar sind, dass sie die mit der Lebenswichtige-Daten-Messeinheit (101) gemessenen ersten lebenswichtigen Daten über ein Kommunikationsnetzwerk (119) senden; und
eine serverseitige Kommunikationseinheit (121), die so betreibbar ist, dass sie die Prädiktionsänderungsmuster über das Kommunikationsnetzwerk sendet,

**dadurch gekennzeichnet, dass**
zumindest die Lebenswichtige-Daten-Messeinheit und die Zeitmesseinheit (102) über jede personenseitige Kommunikationseinheit mit dem Kommunikationsnetzwerk verbunden sind und
zumindest die Referenzinformationen-Speichereinheit (104), die Referenzänderungsmuster-Erzeugungseinheit (115) und die Prädiktionsänderungsmuster-Erzeugungseinheit (105) über die serverseitige Kommunikationseinheit mit dem Kommunikationsnetzwerk verbunden sind.

**5.** Lebenswichtige-Daten-Verwertungssystem nach Anspruch 1, das weiterhin eine Gebührenberechnungseinheit (124) aufweist, die so betreibbar ist, dass sie eine Gebühr berechnet und Gebührenberechnungsinformationen erzeugt, die der Informationsmenge der erzeugten Prädiktionsänderungsmuster entsprechen.

**6.** Lebenswichtige-Daten-Verwertungssystem nach Anspruch 1, das weiterhin eine Anreizberechnungseinheit (125) aufweist, die so betreibbar ist, dass sie personenbezogen einen Anreiz berechnet, und so betreibbar ist, dass sie Anreiz-Informationen erzeugt, die angeben, dass jede Person gemessene lebenswichtige Daten hat.

**7.** Server (120), der ein Lebenswichtige-Daten-Verwertungssystem (100) zum Speichern und Verwerten von gemessenen lebenswichtigen Daten bildet, mit:

einer Personeninformationen-Speichereinheit (103), die so betreibbar ist, dass sie Personeninformationen speichert, die die gemessenen ersten lebenswichtigen Daten und Informationen zu einem Messdatum und einer Messzeit enthalten, an dem / zu der die ersten lebenswichtigen Daten gemessen wurden;
einer Referenzinformationen-Speichereinheit (104), die so betreibbar ist, dass sie die Referenzinformationen speichert, die zweite lebenswichtige Daten und die Informationen zu dem Messdatum und der Messzeit enthalten, an dem / zu der die zweiten lebenswichtigen Daten gemessen wurden;
einer Personenänderungsmuster-Erzeugungseinheit (115), die so betreibbar ist, dass sie aus der Personeninformationen-Speichereinheit die Personeninformationen extrahiert, die einem bestimmten Zeitraum entsprechen, der bis zu dem Messdatum und der Messzeit der ersten lebenswichtigen Daten reicht, die eine erste vorgegebene Bedingung erfüllen, und so betreibbar ist, dass sie aufgrund der extrahierten Personeninformationen ein Personenänderungsmuster erzeugt, wenn die gemessenen ersten lebenswichtigen Daten die erste vorgegebene Bedingung erfüllen;
einer Referenzänderungsmuster-Erzeugungseinheit (116), die so betreibbar ist, dass sie aus der Referenzinformationen-Speichereinheit zweite lebenswichtige Informationen extrahiert, die die erste vorgegebene Bedingung erfüllen, so betreibbar ist, dass sie aus der Referenzinformationen-Speichereinheit die Referenzinformationen extrahiert, die dem bestimmten Zeitraum entsprechen, der bis zu dem Messdatum und der Messzeit reicht, an dem / zu der die zweiten lebenswichtigen Daten gemessen wurden, und so betreibbar ist, dass sie aufgrund der extrahierten Referenzinformationen das Referenzänderungsmuster erzeugt; und
einer Prädiktionsänderungsmuster-Erzeugungseinheit (105), die so betreibbar ist, dass sie das Referenzänderungsmuster mit dem Personenänderungsmuster vergleicht, und so betreibbar ist, dass sie aufgrund der Referenzinformationen, die nach dem Erzeugen des Referenzänderungsmusters gewonnen worden sind, ein Prädiktionsänderungsmuster erzeugt, wenn das Vergleichsergebnis eine zweite vorgegebene Bedingung erfüllt.

**8.** Lebenswichtige-Daten-Verwertungsverfahren zum Speichern und Verwerten von gemessenen lebenswichtigen Daten, mit:

einem Lebenswichtige-Daten-Messschritt zum Messen von ersten lebenswichtigen Daten einer Person;
einem Zeitmessschritt zum Erzeugen von Informationen zu einem Messdatum und einer Messzeit, an dem / zu der die ersten lebenswichtigen Daten gemessen wurden;
einem Personeninformationen-Speicherschritt zum Speichern von Personeninformationen, die die ersten lebenswichtigen Daten und die mit den ersten lebenswichtigen Daten assoziierten Informationen zu dem Messdatum und der Messzeit enthalten, in einer Personeninformationen-Speichereinheit;
einem Referenzinformationen-Speicherschritt zum Speichern der Referenzinformationen, die zweite lebenswichtige Daten und das Messdatum und die Messzeit enthalten, an dem / zu der die zweiten lebenswichtigen Daten gemessen wurden, in einer Referenzinformationen-Speichereinheit;
einem ersten Ermittlungsschritt zum Ermitteln, ob die gemessenen ersten lebenswichtigen Daten die erste vorgegebene Bedingung erfüllen oder nicht;
einem Personenänderungsmuster-Erzeugungsschritt zum Extrahieren der Personeninformationen aus der Personeninformationen-Speichereinheit, die einem bestimmten Zeitraum entsprechen, der bis zu dem Messdatum und der Messzeit der ersten lebenswichtigen Daten reicht, die die erste vorgegebene Bedingung erfüllen, und zum Erzeugen des Personenänderungsmusters aufgrund der extrahierten Personeninformationen, wenn in dem ersten Ermittlungsschritt ermittelt wird, dass die ersten lebenswichtigen Daten die erste vorgegebene Bedingung erfüllen;
einem Referenzänderungsmuster-Erzeugungsschritt zum Extrahieren von zweiten lebenswichtigen Daten aus der Referenzinformationen-Speichereinheit, die die erste vorgegebene Bedingung erfüllen, zum Extrahieren der Referenzinformationen aus der Referenzinformationen-Speichereinheit, die dem bestimmten Zeitraum entsprechen, der bis zu dem Messdatum und der Messzeit reicht, an dem / zu der die zweiten lebenswichtigen Daten gemessen wurden, und zum Erzeugen eines Referenzänderungsmusters; und
einem Prädiktionsänderungsmuster-Erzeugungsschritt zum Vergleichen des Referenzänderungsmusters mit dem Personenänderungsmuster und zum Erzeugen eines Prädiktionsänderungsmusters aufgrund der Referenzinformationen, die nach dem Erzeugen des Referenzänderungsmusters aufgrund der extrahierten Referenzinformationen gewonnen worden sind, wenn das Vergleichsergebnis eine zweite vorgegebene Bedingung erfüllt.

**9.** Lebenswichtige-Daten-Verwertungsprogramm, das einen Computer ein Verfahren zum Speichern und Verwerten von gemessenen lebenswichtigen Daten abarbeiten lässt, mit:

einem Lebenswichtige-Daten-Messschritt zum Messen von ersten lebenswichtigen Daten einer Person;
einem Zeitmessschritt zum Erzeugen von Informationen zu einem Messdatum und einer Messzeit, an dem / zu der die ersten lebenswichtigen Daten gemessen wurden;
einem Personeninformationen-Speicherschritt zum Speichern von Personeninformationen, die die ersten lebenswichtigen Daten und die mit den ersten lebenswichtigen Daten assoziierten Informationen zu dem Messdatum und der Messzeit enthalten, in einer Personeninformationen-Speichereinheit;
einem Referenzinformationen-Speicherschritt zum Speichern der Referenzinformationen, die zweite lebenswichtige Daten und die Informationen zu dem Messdatum und der Messzeit enthalten, an dem / zu der die zweiten lebenswichtigen Daten gemessen wurden, in einer Referenzinformationen-Speichereinheit;
einem ersten Ermittlungsschritt zum Ermitteln, ob die gemessenen ersten lebenswichtigen Daten die erste vorgegebene Bedingung erfüllen oder nicht;
einem Personenänderungsmuster-Erzeugungsschritt zum Extrahieren der Personeninformationen aus der Personeninformationen-Speichereinheit, die einem bestimmten Zeitraum entsprechen, der bis zu dem Messdatum und der Messzeit der ersten lebenswichtigen Daten reicht, die die erste vorgegebene Bedingung erfüllen, und zum Erzeugen des Personenänderungsmusters aufgrund der extrahierten Personeninformationen, wenn in dem ersten Ermittlungsschritt ermittelt wird, dass die ersten lebenswichtigen Daten die erste vorgegebene Bedingung erfüllen;
einem Referenzänderungsmuster-Erzeugungsschritt zum Extrahieren von zweiten lebenswichtigen Daten aus der Referenzinformationen-Speichereinheit, die die erste vorgegebene Bedingung erfüllen, zum Extrahieren der Referenzinformationen aus der Referenzinformationen-Speichereinheit, die dem bestimmten Zeitraum entsprechen, der bis zu dem Messdatum und der Messzeit reicht, an dem / zu der die zweiten lebenswichtigen Daten gemessen wurden, und zum Erzeugen eines Referenzänderungsmusters; und
einem Prädiktionsänderungsmuster-Erzeugungsschritt zum Vergleichen des Referenzänderungsmusters mit dem Personenänderungsmuster und zum Erzeugen eines Prädiktionsänderungsmusters aufgrund der Referenzinformationen, die nach dem Erzeugen des Referenzänderungsmusters aufgrund der extrahierten Referenzinformationen gewonnen worden sind, wenn das Vergleichsergebnis eine zweite vorgegebene Bedingung erfüllt.

## Revendications

**1.** Système d’utilisation de données vitales (100) qui stocke et utilise des données vitales mesurées, comprenant :

une unité de mesurage de données vitales (101) utilisable pour mesurer des premières données vitales d’un sujet ;

une unité de mesurage de temps (102) utilisable pour générer des informations sur une date et une heure de mesurage auxquelles les premières données vitales ont été mesurées ;
une unité de stockage d'informations de sujet (103) utilisable pour stocker des informations de sujet incluant les premières données vitales et les informations sur les date et heure de mesurage associées avec les premières données vitales ;
une unité de stockage d'informations de référence (104) utilisable pour stocker des informations de référence incluant des deuxièmes données vitales et les informations sur les date et heure de mesurage auxquelles les deuxièmes données vitales ont été mesurées ;
une unité de génération de modèle de variation de sujet (115) utilisable pour extraire, à partir de ladite unité de stockage d'informations de sujet, les informations de sujet correspondant à une période spécifique qui dure jusqu'aux date et heure des premières données vitales qui satisfont à une première condition prédéterminée, et utilisable pour générer un modèle de variation de sujet basé sur les informations de sujet extraites, dans le cas où les premières données vitales mesurées par ladite unité de mesurage de données vitales satisfont à la première condition prédéterminée ;
une unité de génération de modèle de variation de référence (116) utilisable pour extraire, à partir de ladite unité de stockage d'informations de référence, les deuxièmes données vitales qui satisfont à la première condition prédéterminée, utilisable pour extraire, à partir de ladite unité de stockage d'informations de référence, les informations de référence correspondant à la période spécifique qui dure jusqu'aux date et heure de mesurage auxquelles les deuxièmes données vitales ont été mesurées, et utilisable pour générer un modèle de variation de référence basé sur les informations de référence extraites ; et
une unité de génération de modèle de variation de prédiction (105), utilisable pour comparer le modèle de variation de référence avec le modèle de variation de sujet, et utilisable pour générer un modèle de variation de prédiction basé sur les informations de référence obtenues après que le modèle de variation de référence ait été généré, dans le cas où le résultat de comparaison satisfait à une deuxième condition prédéterminée.

**2.** Système d'utilisation de données vitales selon la revendication 1, comprenant en outre
une unité d'obtention d'informations d'attribut de sujet (117) utilisable pour obtenir des informations d'attribut de sujet qui sont des informations sur le sujet,
dans lequel ladite unité de stockage d'informations de sujet (103) est en outre utilisable pour stocker les informations d'attribut de sujet, en association avec les informations de sujet, et
ladite unité de stockage d'informations de référence (104) est utilisable pour stocker des informations d'attribut de référence en association avec les informations de référence, les informations d'attribut de référence étant des informations sur un sujet dont les deuxièmes données vitales ont été obtenues, les deuxièmes données vitales étant incluses dans les informations de référence.

**3.** Système d'utilisation de données vitales selon la revendication 2, comprenant en outre
une unité d'attribution d'ordre de priorité (123) utilisable pour attribuer un ordre de priorité à chaque modèle de variation de prédiction généré basé sur chaque modèle de variation de référence, selon le résultat de comparaison de (i) les informations d'attribut de référence associées avec chaque modèle de variation de référence et (ii) les informations d'attribut de sujet, dans le cas où il existe plusieurs modèles de variation de référence qui satisfont à la deuxième condition prédéterminée.

**4.** Système d'utilisation de données vitales selon la revendication 1, comprenant en outre
des unités de communication du côté sujet (112), chacune desquelles est utilisable pour envoyer les premières données vitales mesurées par ladite unité de mesurage de données vitales (101) par l'intermédiaire d'un réseau de communication (119) ; et
une unité de communication du côté serveur (121) utilisable pour envoyer les modèles de variation de prédiction par l'intermédiaire du réseau de communication,
dans lequel ladite unité de mesurage de données vitales et ladite unité de mesurage de temps (102) sont raccordées au réseau de communication par l'intermédiaire de chaque unité de communication du côté sujet, et
au moins ladite unité de stockage d'informations de référence (104), ladite unité de génération de modèle de variation de référence (115) et ladite unité de génération de modèle de variation de prédiction (105) sont raccordées au réseau de communication par l'intermédiaire de ladite unité de communication du côté serveur.

**5.** Système d'utilisation de données vitales selon la revendication 1, comprenant en outre
une unité de charge (124) utilisable pour calculer une charge et générer des informations de charge correspondant à la quantité d'informations des modèles de variation de prédiction générés.

6. Système d'utilisation de données vitales selon la revendication 1, comprenant en outre une unité de calcul d'incitation (125) utilisable pour calculer, sur une base sujet par sujet, une incitation et utilisable pour générer des informations d'incitation indiquant que chaque sujet a mesuré les données vitales.

7. Serveur (120) qui constitue un système d'utilisation de données vitales (100) stockant et utilisant des données vitales mesurées, comprenant :

une unité de stockage d'informations de sujet (103) utilisable pour stocker des informations de sujet incluant des premières données vitales mesurées et les informations sur les date et heure de mesurage auxquelles les premières données vitales ont été mesurées ;
une unité de stockage d'informations de référence (104) utilisable pour stocker des informations de référence incluant des deuxièmes données vitales et les informations sur les date et heure de mesurage auxquelles les deuxièmes données vitales ont été mesurées ;
une unité de génération de modèle de variation de sujet (115) utilisable pour extraire, à partir de ladite unité de stockage d'informations de sujet, les informations de sujet correspondant à une période spécifique qui dure jusqu'aux date et heure des premières données vitales qui satisfont à une première condition prédéterminée, et utilisable pour générer un modèle de variation de sujet basé sur les informations de sujet extraites, dans le cas où les premières données vitales mesurées par ladite unité de mesurage de données vitales satisfont à la première condition prédéterminée ;
une unité de génération de modèle de variation de référence (116) utilisable pour extraire, à partir de ladite unité de stockage d'informations de référence, les deuxièmes données vitales qui satisfont à la première condition prédéterminée, utilisable pour extraire, à partir de ladite unité de stockage d'informations de référence, les informations de référence correspondant à la période spécifique qui dure jusqu'aux date et heure de mesurage auxquelles les deuxièmes données vitales ont été mesurées, et utilisable pour générer un modèle de variation de référence basé sur les informations de référence extraites ; et
une unité de génération de modèle de variation de prédiction (105), utilisable pour comparer le modèle de variation de référence avec le modèle de variation de sujet, et utilisable pour générer un modèle de variation de prédiction basé sur les informations de référence obtenues après que le modèle de variation de référence ait été généré dans le cas où le résultat de comparaison satisfait à une deuxième condition prédéterminée.

8. Procédé d'utilisation de données vitales destiné à stocker et à utiliser des données vitales mesurées, comprenant :

une étape de mesurage de données vitales consistant à mesurer des premières données vitales d'un sujet ;
une étape de mesurage de temps consistant à générer des informations sur les date et heure auxquelles les premières données vitales ont été mesurées ;
une étape de stockage d'informations de sujet consistant à stocker, dans une unité de stockage d'informations de sujet, des informations de sujet incluant les premières données vitales et les informations sur les date et heure de mesurage associées avec les premières données vitales ;
une étape de stockage d'informations de référence consistant à stocker, dans une unité de stockage d'informations de référence, les informations de référence incluant des deuxièmes données vitales et les informations sur les date et heure de mesurage auxquelles les deuxièmes données vitales ont été mesurées ;
une première étape de jugement consistant à juger si oui ou non les premières données vitales mesurées satisfont à la première condition prédéterminée ;
une étape de génération de modèle de variation de sujet consistant à extraire, à partir de l'unité de stockage d'informations de sujet, les informations de sujet correspondant à une période spécifique qui dure jusqu'aux date et heure des premières données vitales qui satisfont à une première condition prédéterminée, et générant le modèle de variation de sujet basé sur les informations de sujet extraites, dans le cas où il est jugé au cours de ladite première étape de jugement que les premières données vitales satisfont à la première condition prédéterminée ;
une étape de génération de modèle de variation de référence consistant à extraire, à partir de l'unité de stockage d'informations de référence, les deuxièmes données vitales qui satisfont à la première condition prédéterminée, à extraire, à partir de l'unité de stockage d'informations de référence, les informations de référence correspondant à la période spécifique qui dure jusqu'aux date et heure de mesurage auxquelles les deuxièmes données vitales ont été mesurées, et à générer un modèle de variation de référence ; et
une étape de génération de modèle de variation de prédiction consistant à comparer le modèle de variation de référence avec le modèle de variation de sujet, et à générer un modèle de variation de prédiction basé sur les informations de référence obtenues après que le modèle de variation de référence ait été généré sur la base des informations de référence extraites, dans le cas où le résultat de comparaison satisfait à une deuxième condition prédéterminée.

**9.** Programme d'utilisation de données vitales conduisant un ordinateur à exécuter un procédé destiné à stocker et à utiliser des données vitales mesurées, le programme comprenant :

une étape de mesurage de données vitales consistant à mesurer des premières données vitales d'un sujet ;
une étape de mesurage de temps consistant à générer des informations sur les date et heure auxquelles les premières données vitales ont été mesurées ;
une étape de stockage d'informations de sujet consistant à stocker, dans une unité de stockage d'informations de sujet, des informations de sujet incluant les premières données vitales et les informations sur les date et heure de mesurage associées avec les premières données vitales ;
une étape de stockage d'informations de référence consistant à stocker, dans une unité de stockage d'informations de référence, les informations de référence incluant des deuxièmes données vitales et les informations sur les date et heure de mesurage auxquelles les deuxièmes données vitales ont été mesurées ;
une première étape de jugement consistant à juger si oui ou non les premières données vitales mesurées satisfont à la première condition prédéterminée ;
une étape de génération de modèle de variation de sujet consistant à extraire, à partir de l'unité de stockage d'informations de sujet, les informations de sujet correspondant à une période spécifique qui dure jusqu'aux date et heure des premières données vitales qui satisfont à une première condition prédéterminée, et générant le modèle de variation de sujet basé sur les informations de sujet extraites, dans le cas où il est jugé au cours de ladite première étape de jugement que les premières données vitales satisfont à la première condition prédéterminée ;
une étape de génération de modèle de variation de référence consistant à extraire, à partir de l'unité de stockage d'informations de référence, les deuxièmes données vitales qui satisfont à la première condition prédéterminée, à extraire, à partir de l'unité de stockage d'informations de référence, les informations de référence correspondant à la période spécifique qui dure jusqu'aux date et heure de mesurage auxquelles les deuxièmes données vitales ont été mesurées, et à générer un modèle de variation de référence ; et
une étape de génération de modèle de variation de prédiction consistant à comparer le modèle de variation de référence avec le modèle de variation de sujet, et à générer un modèle de variation de prédiction basé sur les informations de référence obtenues après que le modèle de variation de référence ait été généré sur la base des informations de référence extraites, dans le cas où le résultat de comparaison satisfait à une deuxième condition prédéterminée.

FIG. 1

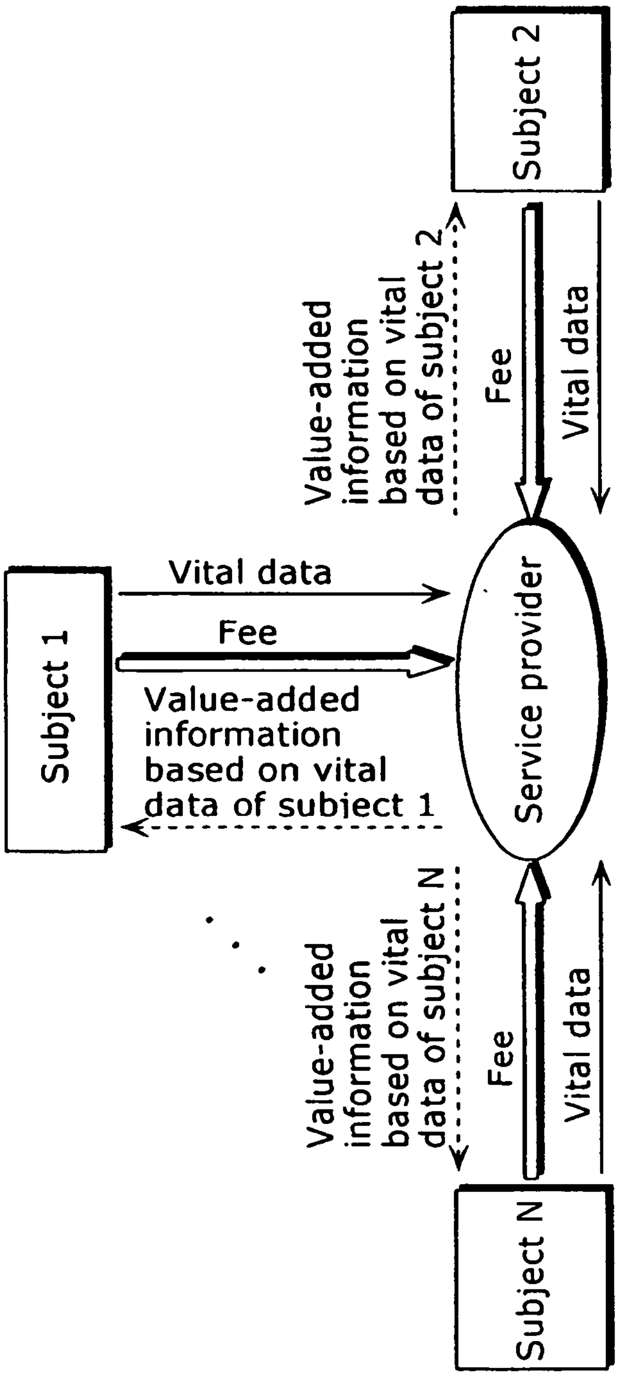
Subject 1
Vital data
Fee
Value-added information based on vital data of subject 1
Service provider
Subject 2
Value-added information based on vital data of subject 2
Fee
Vital data
Subject N
Value-added information based on vital data of subject N
Fee
Vital data

FIG. 2

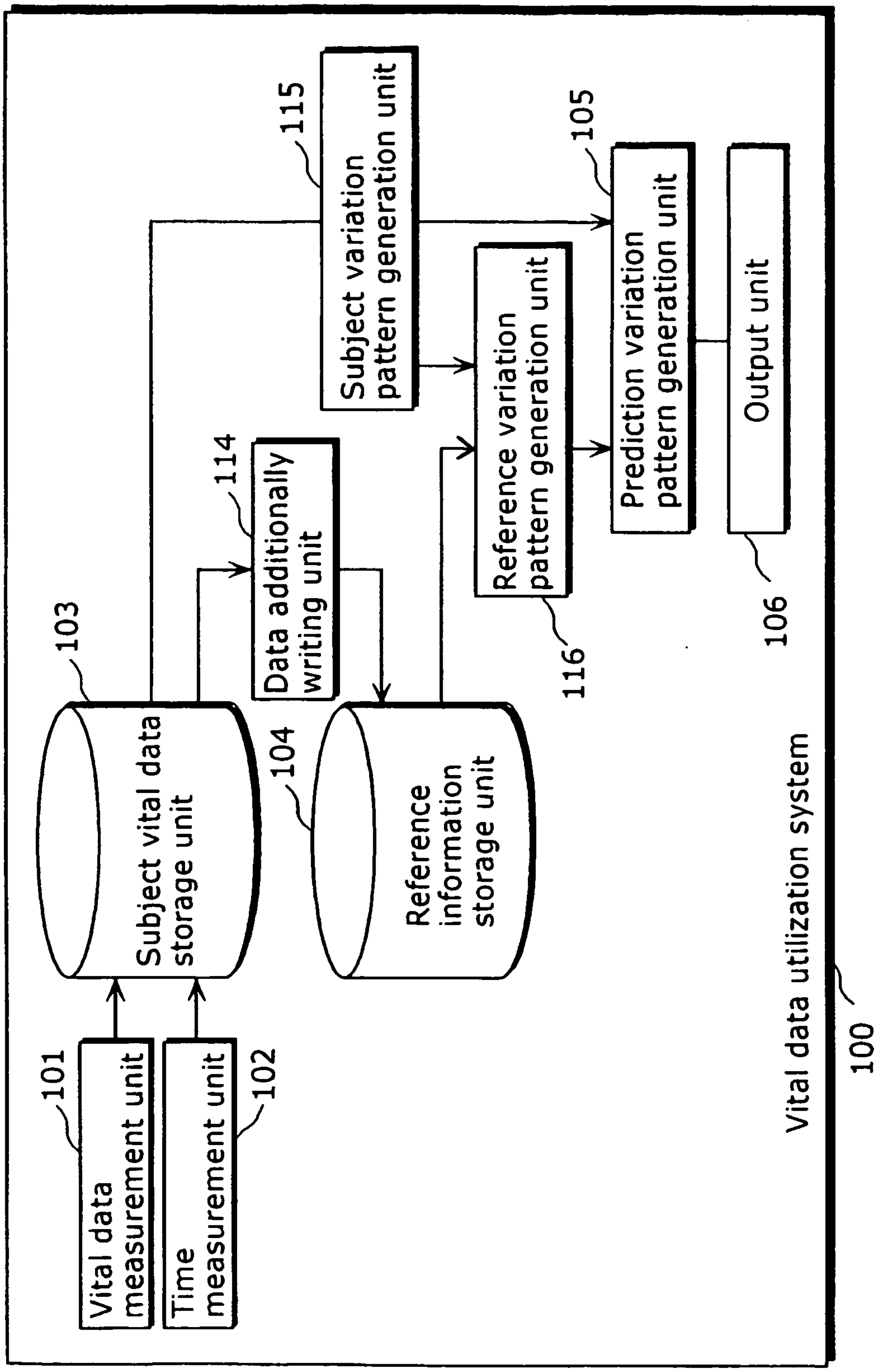
Vital data measurement unit
101
Time measurement unit
102
Subject vital data storage unit
103
Reference information storage unit
104
Data additionally writing unit
114
Subject variation pattern generation unit
115
Reference variation pattern generation unit
116
Prediction variation pattern generation unit
105
Output unit
106
Vital data utilization system
100

FIG. 3

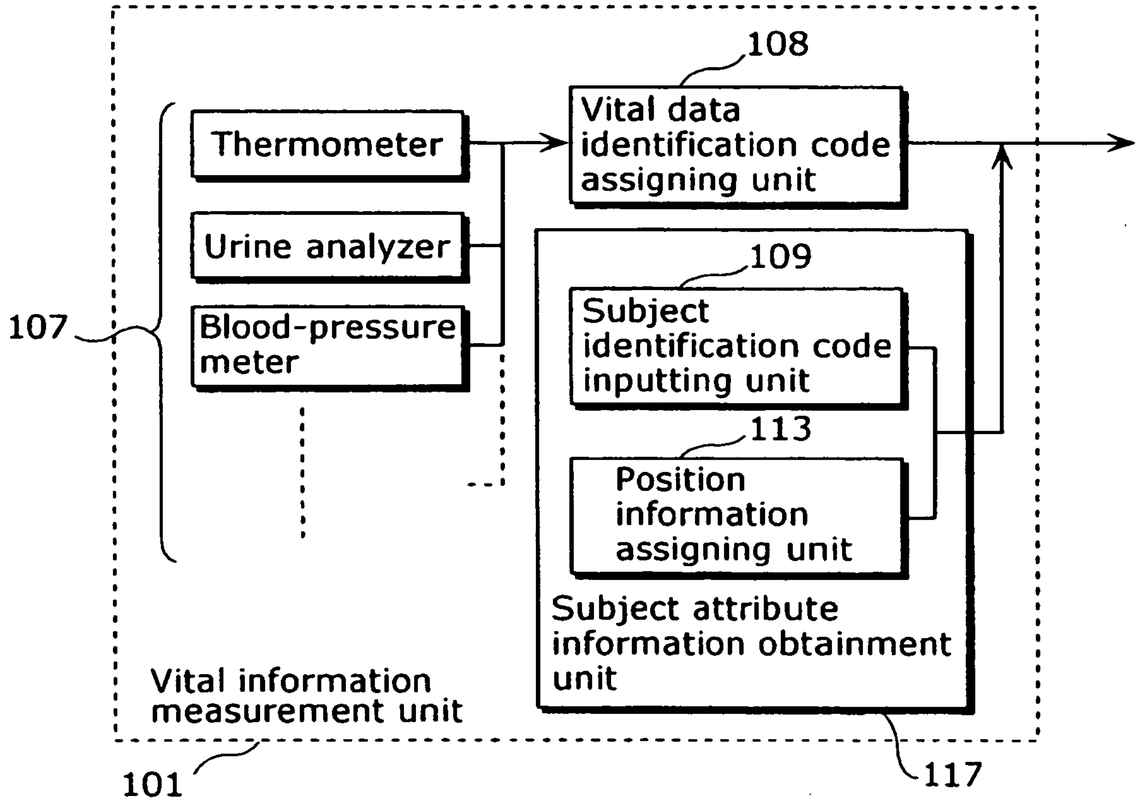
108
Vital data identification code assigning unit
Thermometer
Urine analyzer
Blood-pressure meter
107
109
Subject identification code inputting unit
113
Position information assigning unit
Subject attribute information obtainment unit
Vital information measurement unit
101
117

FIG. 4

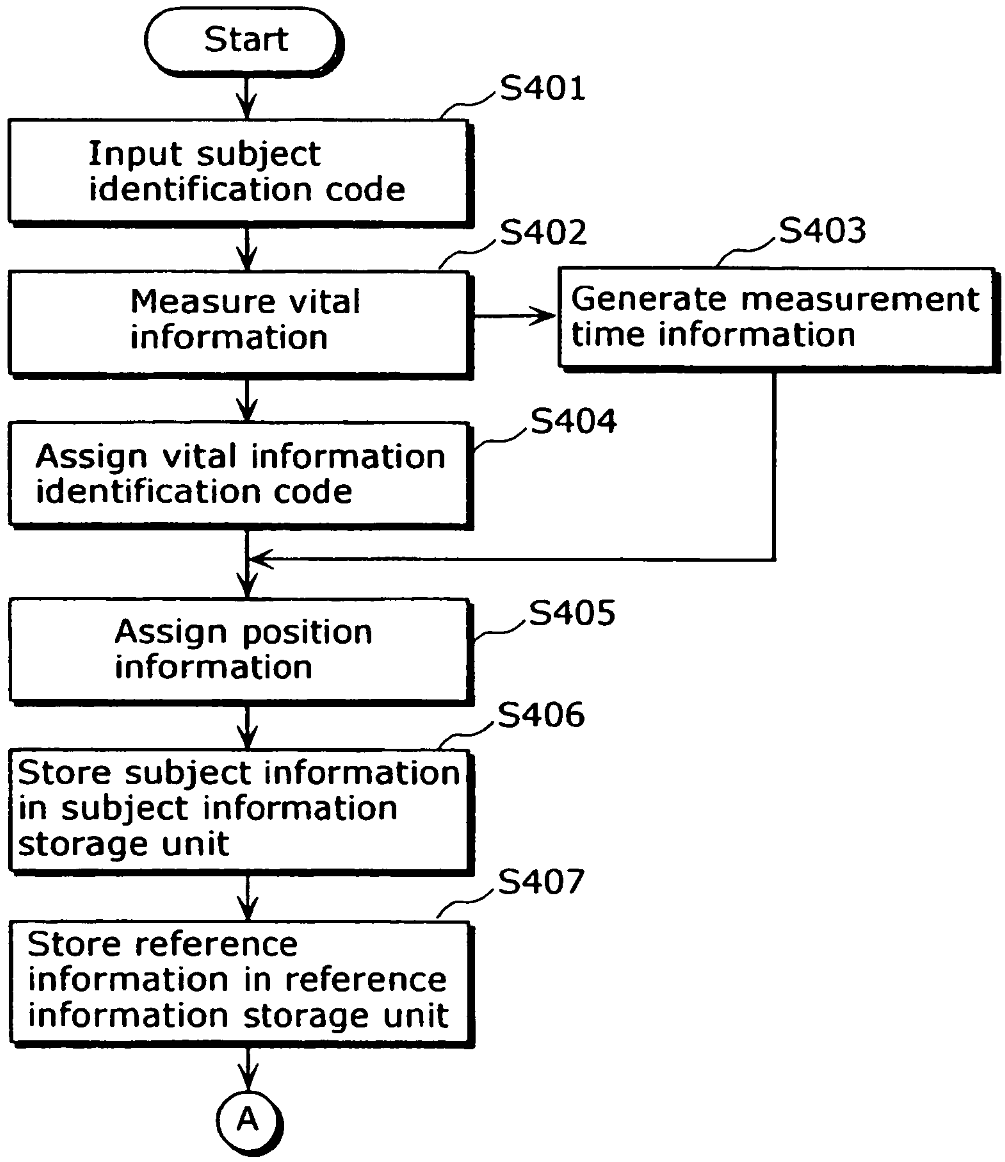
Start
S401
Input subject identification code
S402
Measure vital information
S403
Generate measurement time information
S404
Assign vital information identification code
S405
Assign position information
S406
Store subject information in subject information storage unit
S407
Store reference information in reference information storage unit
A

FIG. 5

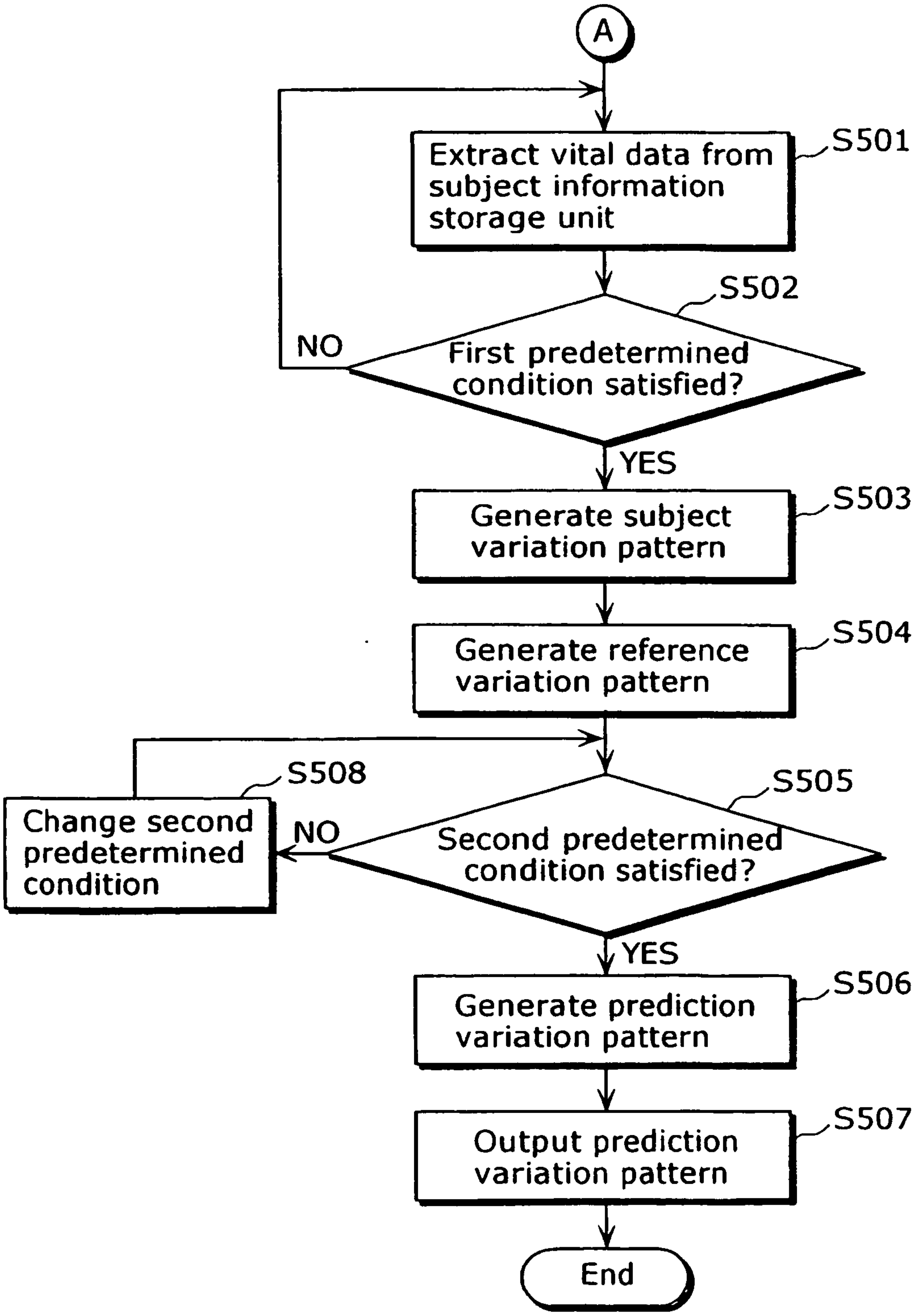
A
Extract vital data from subject information storage unit
S501
First predetermined condition satisfied?
S502
NO
YES
Generate subject variation pattern
S503
Generate reference variation pattern
S504
Second predetermined condition satisfied?
S505
NO
Change second predetermined condition
S508
YES
Generate prediction variation pattern
S506
Output prediction variation pattern
S507
End

FIG. 6

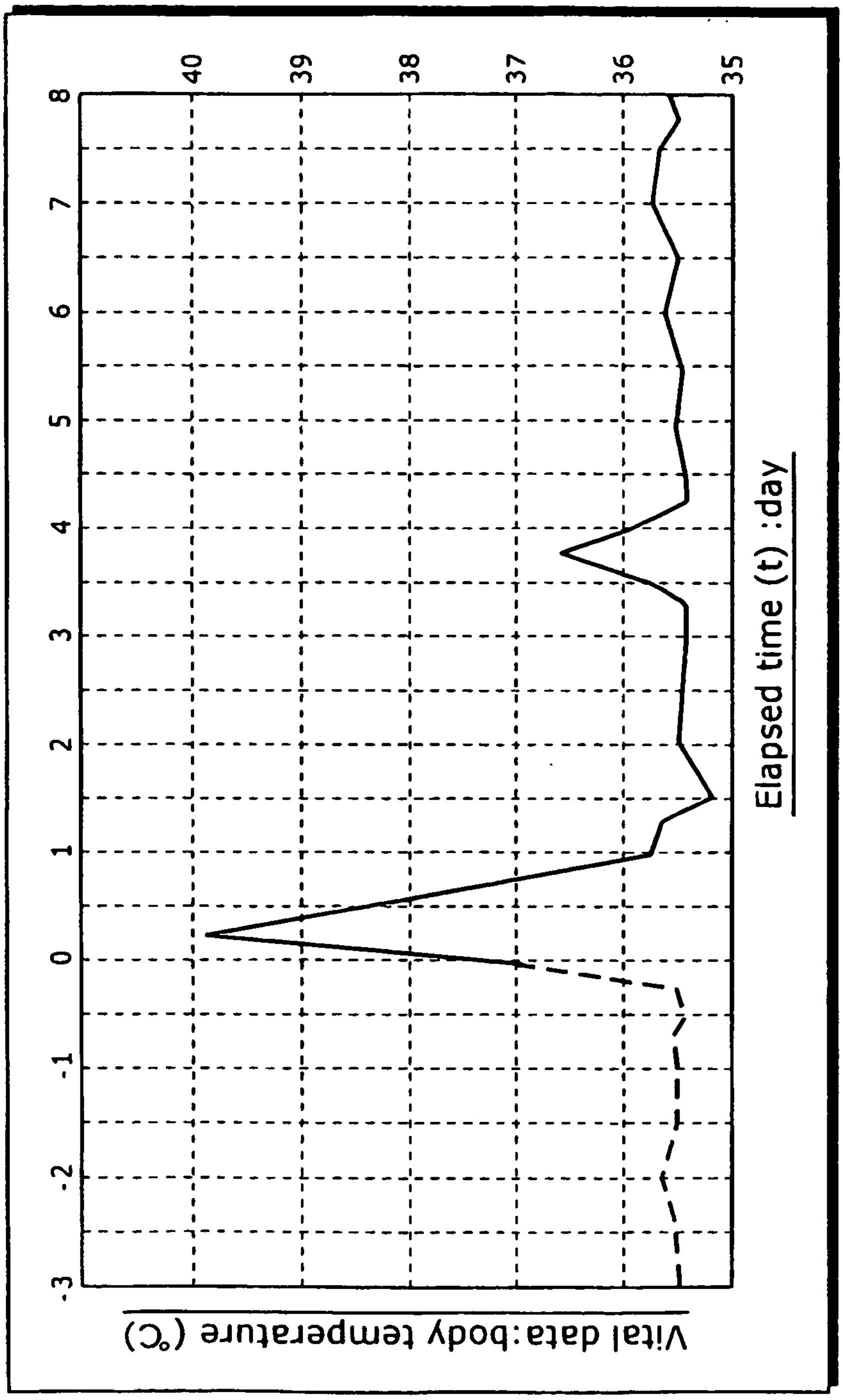
Vital data:body temperature (°C)
40
39
38
37
36
35
-3
-2
-1
0
1
2
3
4
5
6
7
8
Elapsed time (t) :day

FIG. 7

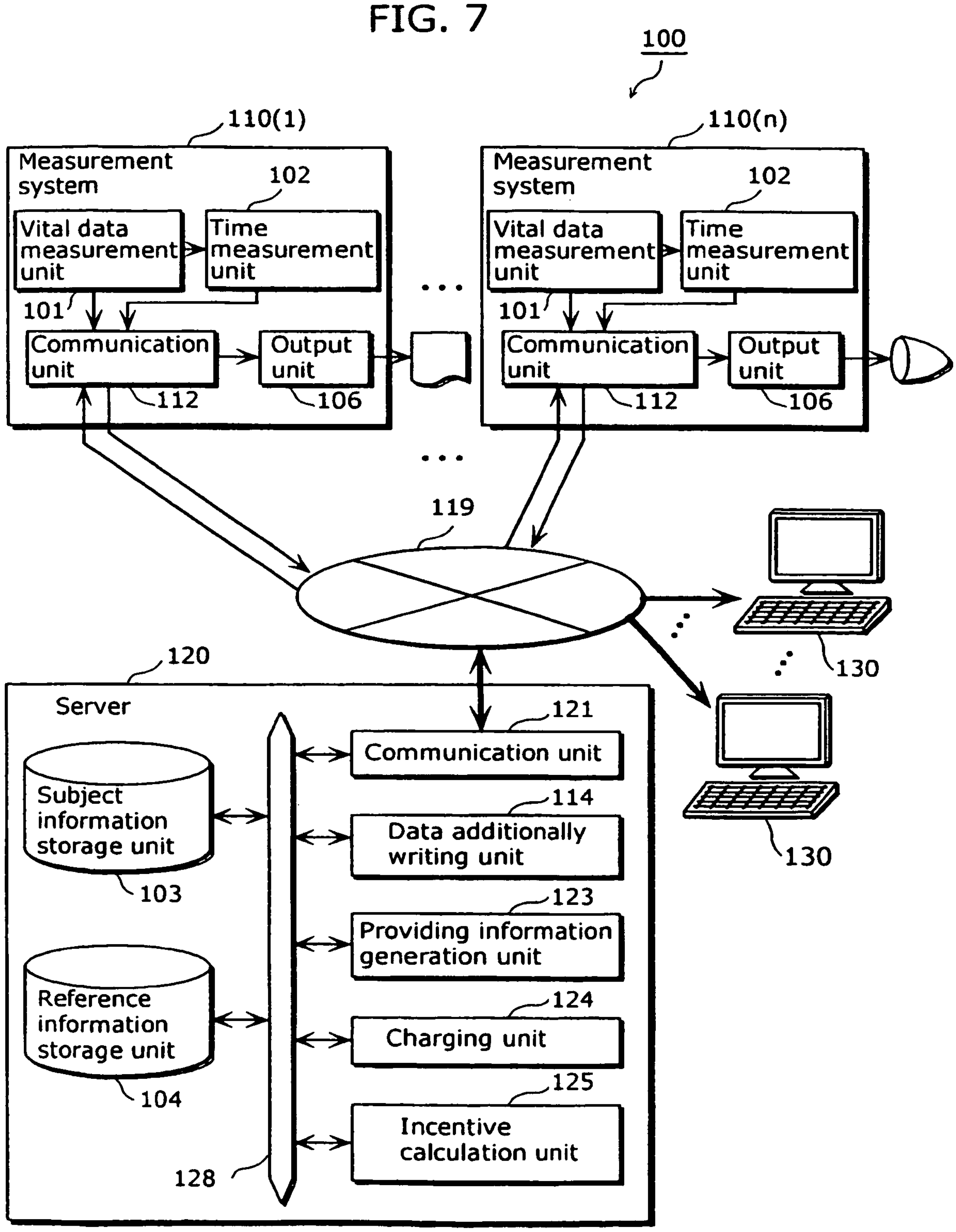
100
110(1)
Measurement system
102
Vital data measurement unit
Time measurement unit
101
Communication unit
Output unit
112
106
110(n)
Measurement system
102
Vital data measurement unit
Time measurement unit
101
Communication unit
Output unit
112
106
119
120
Server
121
Communication unit
Subject information storage unit
103
114
Data additionally writing unit
123
Providing information generation unit
Reference information storage unit
104
124
Charging unit
125
Incentive calculation unit
128
130
130

FIG. 8

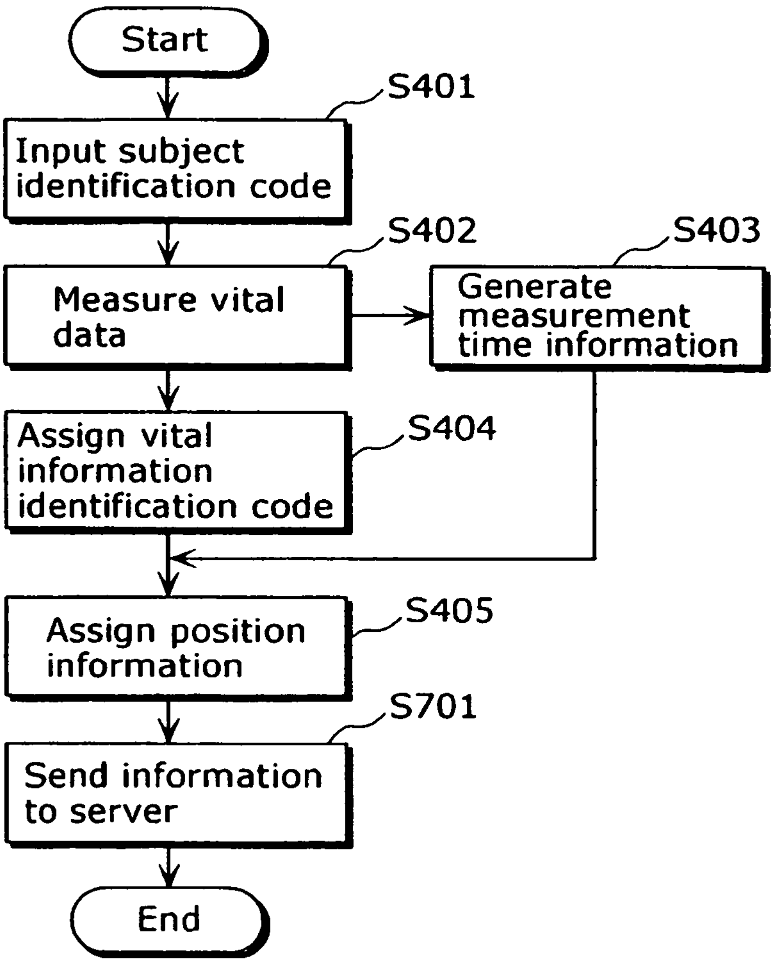
Start
S401
Input subject identification code
S402
Measure vital data
S403
Generate measurement time information
S404
Assign vital information identification code
S405
Assign position information
S701
Send information to server
End

FIG. 9

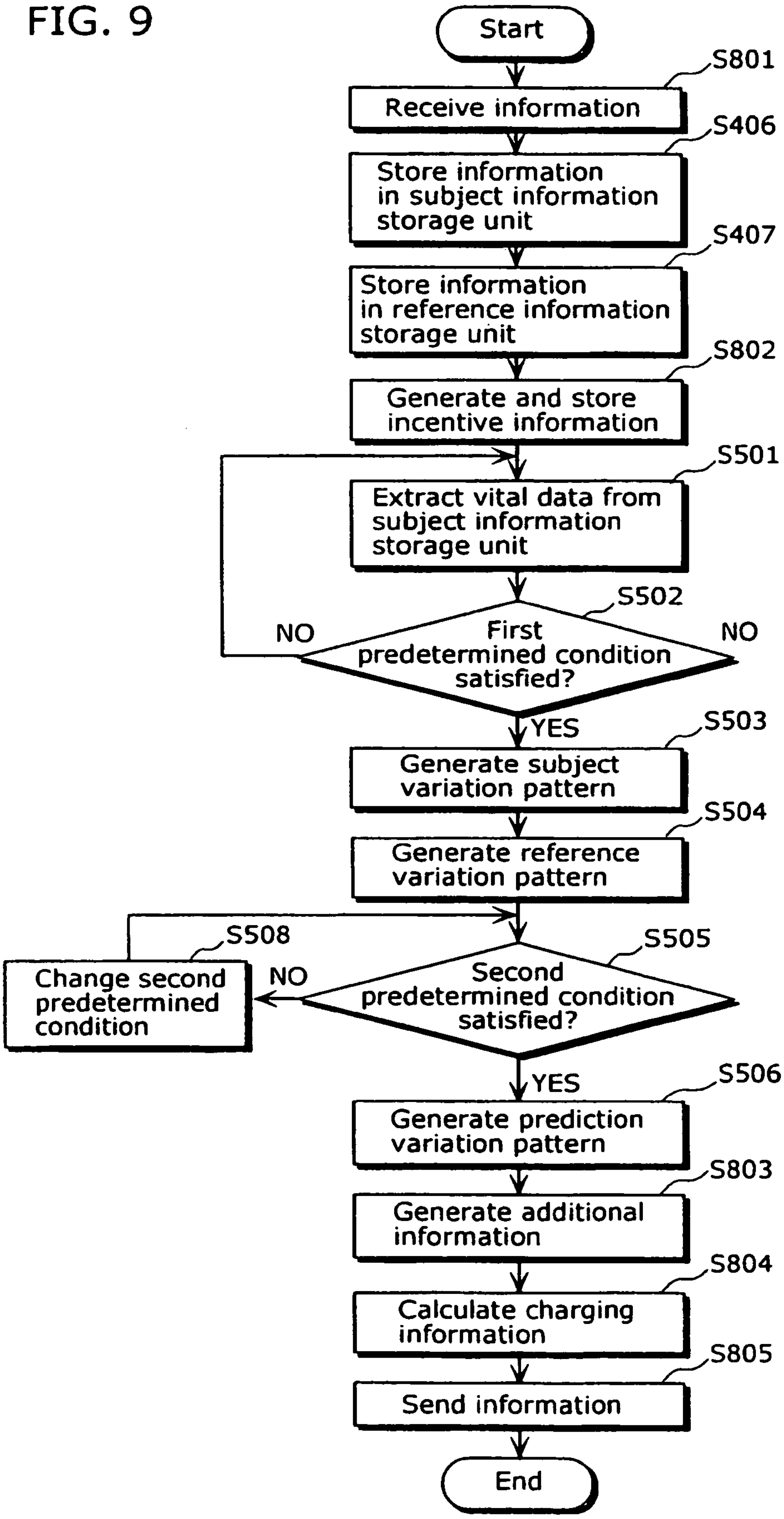
Start
S801
Receive information
S406
Store information in subject information storage unit
S407
Store information in reference information storage unit
S802
Generate and store incentive information
S501
Extract vital data from subject information storage unit
S502
First predetermined condition satisfied?
NO
NO
YES
S503
Generate subject variation pattern
S504
Generate reference variation pattern
S505
Second predetermined condition satisfied?
NO
S508
Change second predetermined condition
YES
S506
Generate prediction variation pattern
S803
Generate additional information
S804
Calculate charging information
S805
Send information
End

FIG. 10

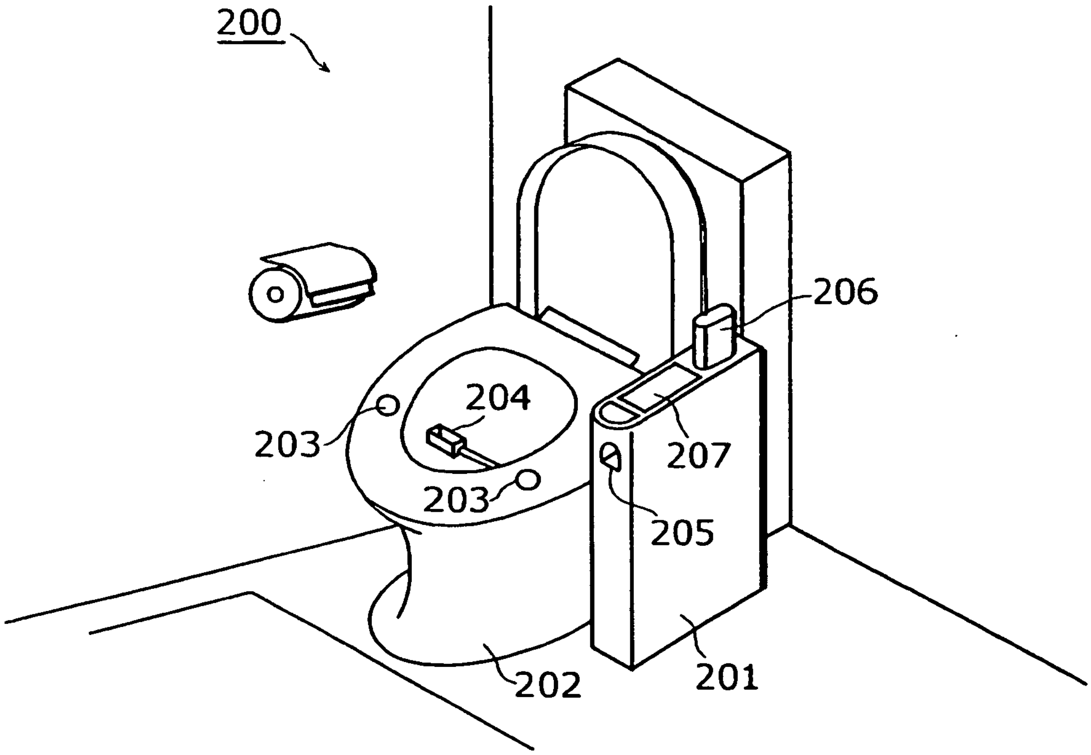

FIG. 11

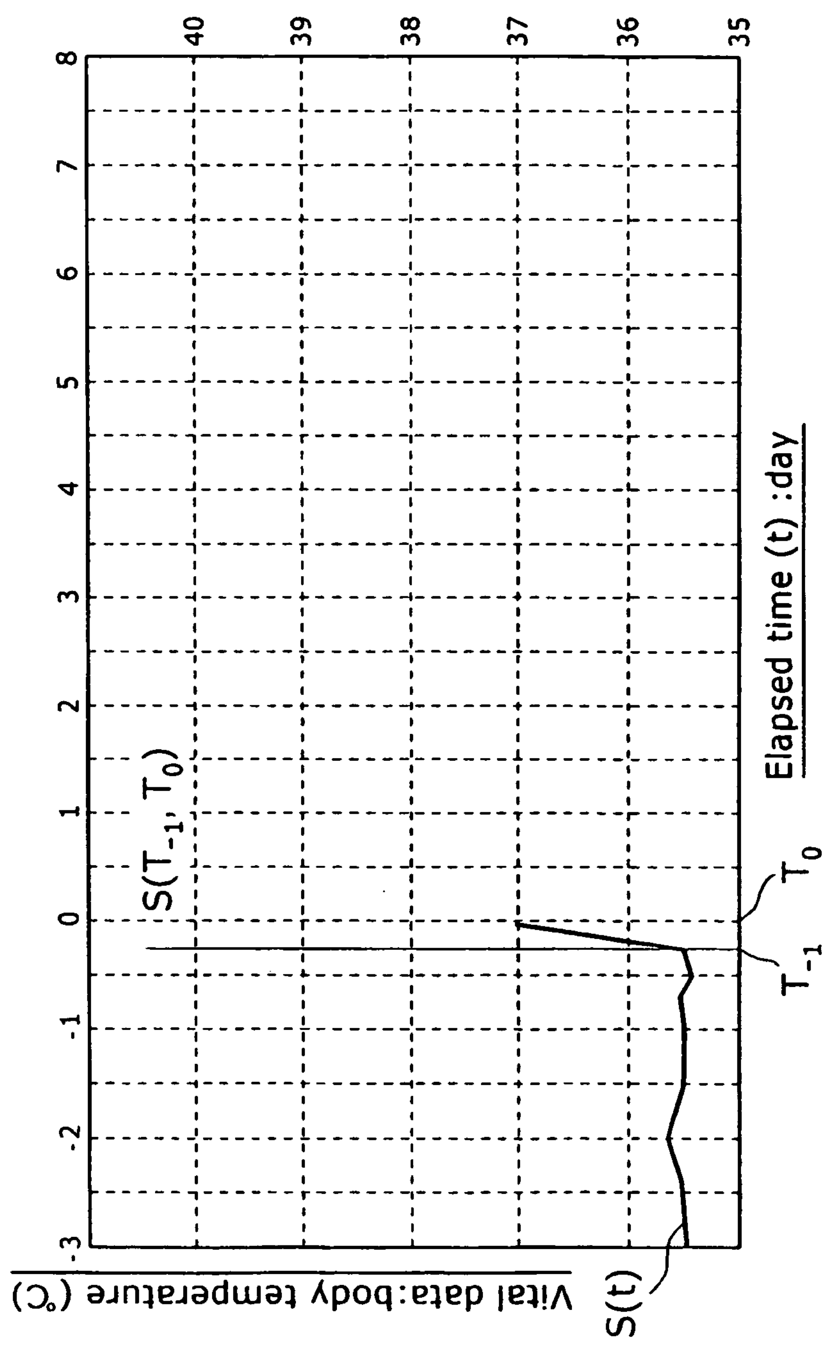
Vital data:body temperature (°C)
40
39
38
37
36
35
Elapsed time (t) :day
-3
-2
-1
0
1
2
3
4
5
6
7
8
S(T-1, T0)
S(t)
T-1
T0

FIG. 12

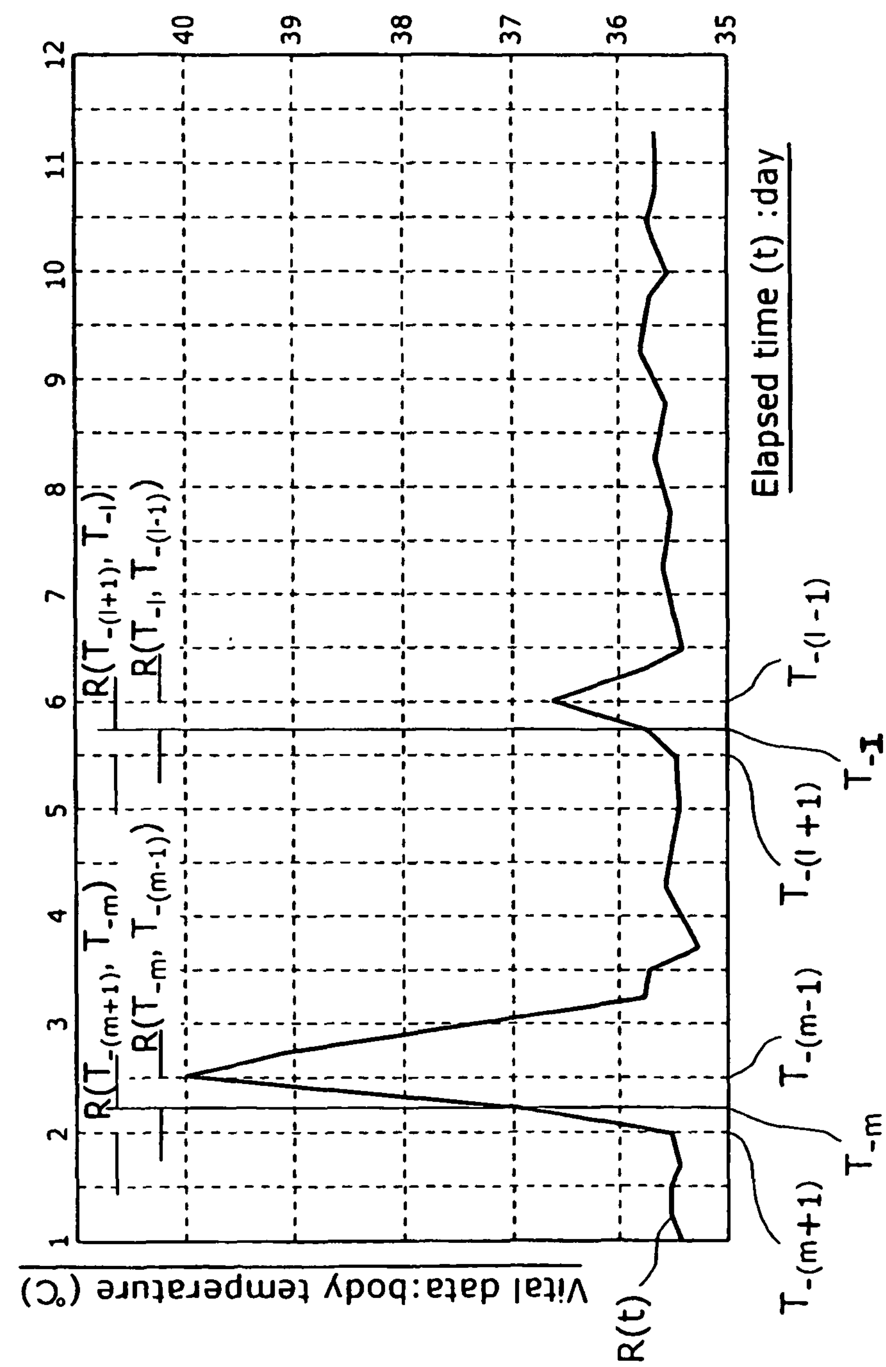
Vital data:body temperature (°C)
Elapsed time (t) :day
R(t)
$R(T_{-(m+1)}, T_{-m})$
$R(T_{-m}, T_{-(m-1)})$
$R(T_{-(l+1)}, T_{-l})$
$R(T_{-l}, T_{-(l-1)})$
$T_{-(m+1)}$
$T_{-m}$
$T_{-(m-1)}$
$T_{-(l+1)}$
$T_{-l}$
$T_{-(l-1)}$
1 2 3 4 5 6 7 8 9 10 11 12
35 36 37 38 39 40

FIG. 13

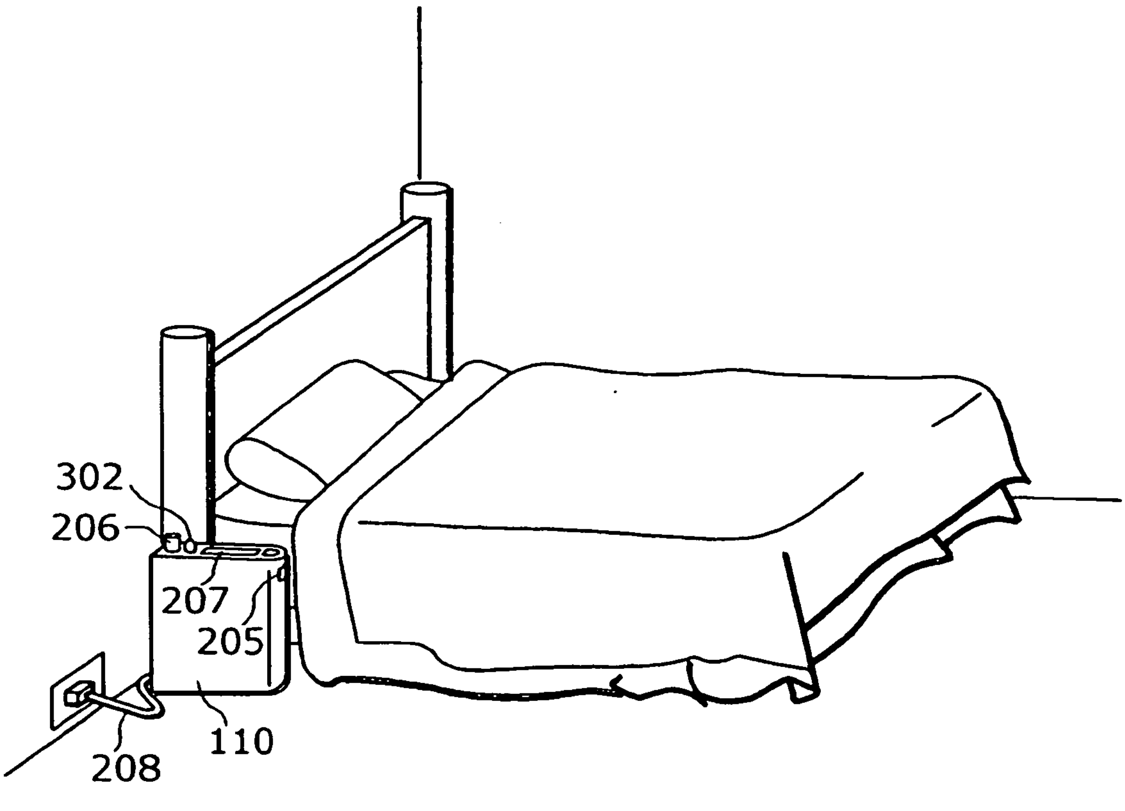

FIG. 14

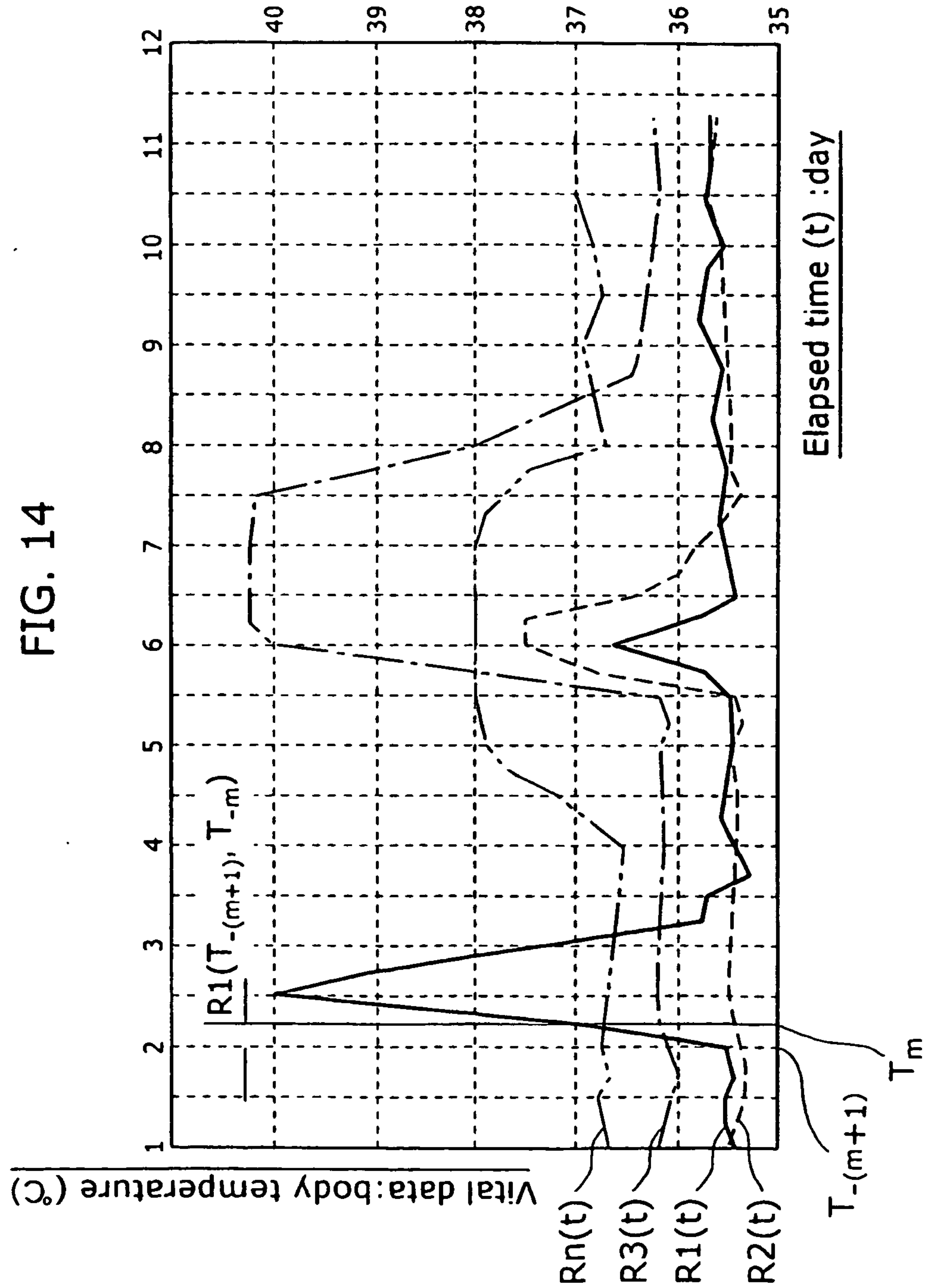
R1(T-(m+1), T-m)
Rn(t)
R3(t)
R1(t)
R2(t)
T-(m+1)
Tm
Elapsed time (t) :day
Vital data:body temperature (°C)
1
2
3
4
5
6
7
8
9
10
11
12
35
36
37
38
39
40

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2003125612 A
- JP 2001137199 A